# EUROPEAN PATENT APPLICATION

(11) **EP 4 008 427 A1**
(43) Date of publication of application: **08.06.2022**
(21) Application number: 20212188.5
(22) Date of filing: 07.12.2020
(51) Int. Cl.: B01J 13/04, B01J 13/08, B01J 13/20, A61K 47/58

(54) **PROGRAMMABLE POLYMER DROPLETS AND ASSOCIATED USES**

(71) Applicant: ETH Zurich, 8092 Zurich (CH)
(72) Inventor: AROSIO, Paolo, 8046 Zürich (CH); KOPP, Marie, 8044 Zürich (CH); CAPASSO PALMIERO, Umberto, 8049 Zürich (CH); PAGANINI, Carolina, 8005 Zürich (CH)

(57) **Abstract**

The present invention pertains to liquid droplets comprising a polymer according to Formula (I) and to their use in an aqueous medium for the controlled uptake of compounds from outside the droplets into the droplets for separation, storage and/or reaction of compounds inside the droplets, as well as associated methods of production of the polymer.

## Description

The present invention pertains to liquid droplets comprising a polymer according to Formula (I) and to their use in an aqueous medium for the controlled uptake of compounds from outside the droplets into the droplets for separation, storage and/or reaction of compounds inside the droplets.

The bioseparation and detection of biomolecules in complex media can require laborious and expensive procedures. The selective capture of biomolecules in complex media is an important aspect both in the development of biomedical detection assays and in the purification of biopharmaceuticals. The biopurification, bioseparation and detection of analytes is commonly performed by either using solid resins or thermoresponsive polymers functionalized with targeting agents. Relevant examples include PNIPAM, peptide polymers and condensed phases obtained via simple or complex coacervation (see, e.g., I.N. Savina, et al., Chapter 15, Smart Polymers and their Applications (Second Edition), Woodhead Publishing, 2019, Pages 533-565; Rahul D. Sheth et al., Journal of Biotechnology, 192, Part A, 2014, 11-19; N. Shimada et al., Biomacromolecules 2013, 14, 5, 1452-1457; J. van Lente et al., Biomacromolecules 2019, 20, 10, 3696-3703). However, these solid or solid-like materials do not have the ability to renew their interface and, thus, present lower concentration efficacies. In addition, they encode multiple interactions which are not able to completely exclude solutes from complex mixture. For this reason, they require multiple washing steps to remove all the unspecific solutes that are retained during the separation process. This aspect can challenge their application for the detection of pathological biomarkers and the purification of biomolecules.

It is the objective of the present invention to provide materials with utility in purification, separation and detection of compounds in liquid environments.

In a first aspect, the present invention is directed to a use of liquid droplets in an aqueous medium for the controlled uptake of compounds from outside the droplets into the droplets for separation, storage and/or reaction of compounds inside the droplets, wherein the droplets comprise a polymer according to Formula (I) 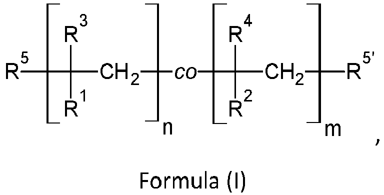 wherein
**n** is an integer selected from 0 to 2500, optionally 1 or 2 to 2500, optionally from 25 to 250;
**m** is an integer selected from 2 to 2500, optionally from 25 to 250;
**R³** and **R⁴** are each independently selected from the group consisting of hydrogen and methyl;
**R⁵** and **R^{5'}** are independently selected from
   - hydrogen, linear or branched (C₁₋₁₀)alkyl and phenyl;
   - a group resulting from a polymerization initiator, optionally a group resulting from a RAFT polymerization initiator, optionally 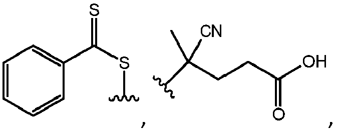 or
   - a target binding moiety (TBM), optionally a TBM attached to or reacted with a group resulting from a polymerization initiator, optionally from a RAFT polymerization initiator, optionally 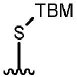 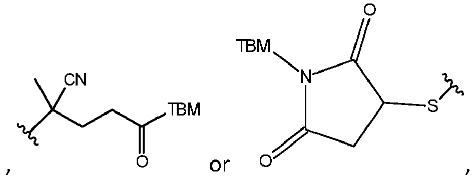 wherein the TBM is optionally selected from the group consisting of azide, alkyne, carboxylate, amine, thiol, hydroxyl, aldehyde, cyanate, sulfonyl, tosyl, tresyl, epoxide, carbonate, halide, anhydride, carbamate, imidazole, azlactone, triazine, maleimide, aziridine, peroxide, acyl, anthraquinone, diazo, diazirine, psoralen, NHS ester, imido ester, (C₂₋₁₀)alkenyl or alkyl groups, DBCO, cytosine, guanidine, streptavidin, avidin, biotin, an aptamer, a peptide, a protein, an oligonucleotide, optionally a hydrophilic uncharged oligopolymer, a positively or negatively charged oligopolymer, a nucleic acid, a carbohydrate, a dye, a cell, an antibody, an organic and inorganic nanoparticle, hydrophilic uncharged oligopolymers, positively and negatively charged oligopolymers;
**R¹** is selected from the group consisting of
   (i) -X-hydrogen, -X-methyl, -X-ethyl and -X-propyl; 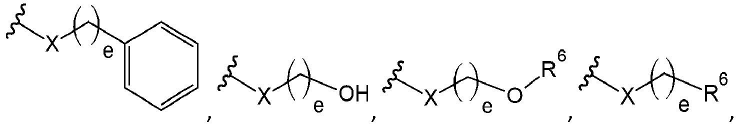
   (ii) 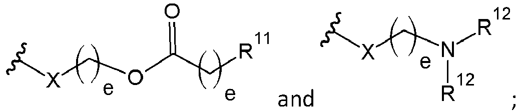
   (iii) 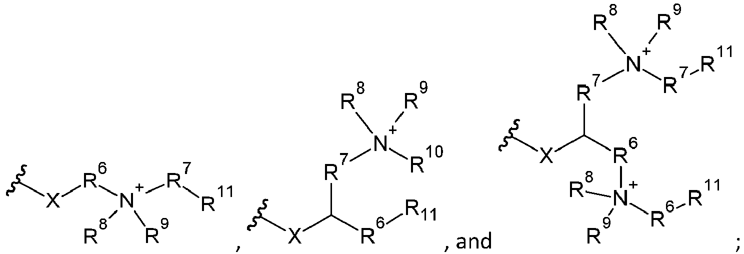
   (iv) 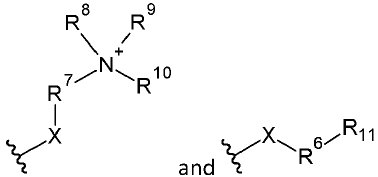 in a ratio of about 1:1 within the polymer;
   (v) 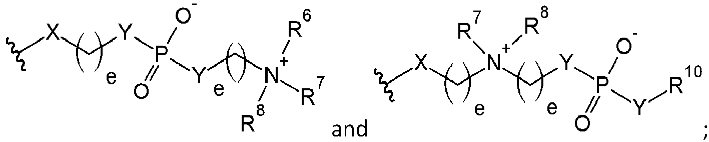 and
   (vi) 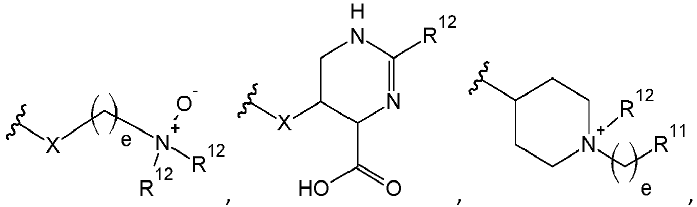 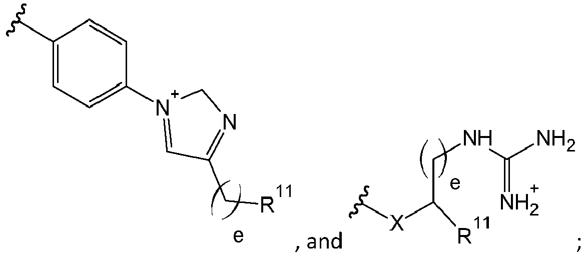
**R²** is selected from the group consisting of 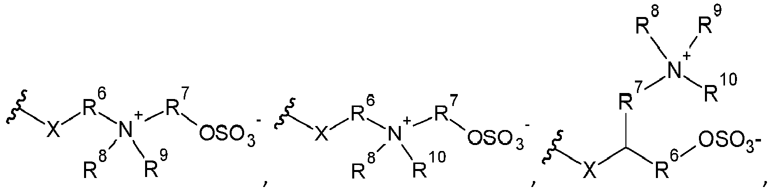 and 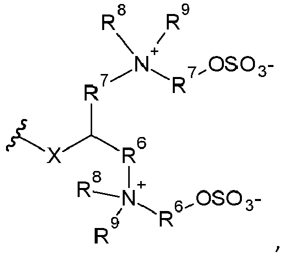 wherein
   **X** is selected from the group consisting of -C(=O)-O-, -C(=O)-NH-, aliphatic and aromatic carbo- or heterocycle;
   **Y** is oxygen or absent;
   **e** is an integer selected from 1 to 5, optionally from 2 to 3;
   **R⁶** and **R⁷** are each independently selected from the group consisting of
      linear or branched, substituted or non-substituted (C₁₋₁₀)alkyl, optionally (C₁₋₅)alkyl, optionally methyl, ethyl and propyl,
      linear or branched, substituted or non-substituted (C₁₋₁₀)alkyl, optionally (C₂₋₆)alkyl chains, comprising one or more heteroatoms selected from the group consisting of O, N, S and P, optionally (C₂₋₅)alkyl ethers,
      (C₂₋₁₀)alkenyl,
      phenyl, and an aromatic heterocycle, optionally pyridine, pyrrole, furan and imidazole;
   **R⁸, R⁹** and **R¹⁰** are each independently selected from the group consisting of hydrogen, linear or branched, substituted or non-substituted (C₁₋₁₀)alkyl, optionally (C₁₋₅)alkyl, optionally methyl, ethyl and propyl, (C₂₋₁₀)alkenyl, phenyl, an aromatic heterocycle, optionally pyridine, pyrrole, furan and imidazole, 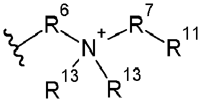 when forming R¹, 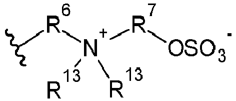 when forming R²;
   **R⁸** and **R⁹** together or two **R¹³** together optionally form an aliphatic or aromatic heterocycle, optionally a piperidine ring, a piperazine ring or a morpholine ring;
   **R⁸, R⁹** and **R⁶** or **R⁸, R⁹** and **R⁷** optionally together form a five or six-membered ring, optionally a pyridine ring, a piperidine ring, pyrrole ring, a pyrimidine ring, a pyrazole ring, an imidazole ring, a pyrazine ring, an isoxazole ring or an oxazole ring; wherein one of **R⁸** or **R⁹** is absent if **R⁸** and **R⁶**, **R⁹** and **R⁶** or **R⁸** and **R⁷,** or **R⁹** and **R⁷** together form an aromatic ring;
   **R¹¹** is selected from the group consisting of -SO₃⁻, -C(=O)O⁻, and 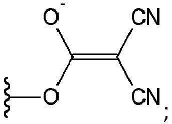
   **R¹²** is selected from the group consisting of methyl, ethyl and propyl;
   **R¹³** is independently selected from the group consisting of linear or branched, substituted or non-substituted (C₁₋₁₀)alkyl, optionally (C₁₋₅)alkyl, optionally methyl, ethyl and propyl, (C₂₋₁₀)alkenyl, phenyl, an aromatic heterocycle, optionally pyridine, pyrrole, furan and imidazole,
   wherein the polymer optionally is crosslinked by a crosslinker, optionally a crosslinker as R¹.

All definitions, examples and explanations provided herein are valid for all aspects and embodiments of the present invention if not specifically indicated otherwise.

The droplets comprising the polymer for use as described herein for all aspects and embodiments are liquid in an aqueous medium, which means that they do not form solids but are coacervates with an essentially spherical shape, e.g. of about 3 to 100 µm in diameter, that are able to coalesce, e.g. to form larger droplets or a continuous phase. Moreover, and for example, the droplets exhibit recovery in order of seconds in fluorescence recovery after photobleaching (FRAP) experiments.

The polymers of the droplets described herein for all aspects and embodiments are able to encode electrostatic attractive interactions, leading to the formation of the liquid droplets with many relevant features, for example: i) the droplets present low interfacial tension and, for this reason, generally do not require a high amount of energy to be generated; ii) the droplets can reversibly assemble and disassemble upon changes in the surrounding environment (e.g. by changing either temperature or ionic strength); and iii) the droplets can avoid unspecific protein adsorption on their surface and preferentially exclude many solutes. All these features are important when using these droplets for isolation of specific components in a mixture. When functionalized with a targeting agent (e.g. antibodies, biotin, etc. which can be easily conjugated to the polymer as R⁵ and/or R^{5'}), the droplets can selectively interact with the biomolecule of interest and preferentially exclude all other components. The droplets can be therefore used to, e.g., upconcentrate and separate only the desired compound from complex mixtures. Also, the targeting agent can aid in an upconcentration which goes beyond the statistical distribution of targeted compounds. The liquid nature of the droplet and the spherical shape (i.e. high surface to volume ratio) are important aspects to increase the solute upconcentratrion due to the presence of a dynamic interface. Moreover, the possibility to choose different targeting agents confers modularity and flexibility. Additionally, the technology involving the droplets described herein is also easily scalable.

Thanks to these properties, these smart synthetic droplets can be used in many applications, in particular for diagnostics and for the purification of biopharmaceuticals. In the case of diagnostics, these droplets are able to, e.g., preferentially recruit pathological biomarkers from complex fluids and to detect them without the need of further purification steps, in sharp contrast with the methods developed so far (e.g. magnetic beads or ELISA). In fact, the droplets act as signal concentrator. In addition, the present invention can be easily implemented in microfluidics devices allowing the use of small amounts of samples and the increase of the solute concentration factor, e.g. at least up to 10⁵. This is an aspect not achievable at bulk scale with state of the art devices. The droplets can also be used as pre-isolation tool for further processing in the context of biomarker detection for disease diagnosis.

Based on pre-designed polymer solubility properties, the change in solubility of the polymer for use as droplets described herein, upon target binding can also be used to detect the presence of target compounds which results in the shrinkage of the droplets and allows the measurement of target compounds without requiring any secondary labeling step. A first target application can be the field of disease diagnosis. The droplets of the present invention could also be used to measure binding affinities, which would be of high relevance for instance for drug discovery, where little amount of candidate biotherapeutics are available, and are usually not purified and therefore need to be characterized directly in their complex environment.

The droplets could further be used as tissue engineering scaffolds. A possible application would be scaffolds for cell culture growth. Once cell clusters reach a defined size, issues may arise inside the cluster to transport essential metabolites to the most inner cells at the core of the cluster, to remove toxic metabolites and transport oxygen. If constant environment is not guaranteed, this can lead to cell death, or cell differentiation in the case of stem cell cultures. Here, the unique properties of the liquid droplets described herein can be used to induce spontaneous or activated splitting of the the cell cluster once it has reached a critical size in order to ensure sufficient mass transport and a constant microenvironment during cell culture growth.

In the case of purification of biopharmaceuticals, the liquid droplets can selectively recover the product from complex media and quickly elute it by applying an external stimulus, e.g. a change in the ionic strength or pH. This technology is scalable and able to gently and rapidly purify small and large bioproducts.

For drug discovery applications, the liquid droplets can be functionalized with the desired target, and binding libraries of e.g. antibodies or aptamers or other binding agents can be tested. The binding affinity between the target and the binding agent can be measured by monitoring the change in droplet properties upon binding, such as signal increase in the droplet due to the selective uptake of the binding agent, or changes in solubility properties upon binding measured by the Tcp, or the decrease in droplet size. A key advantage is that the preferential exclusion of the droplet material allows to perform these operations in complex mixtures, such as cell lysates, or partially purified binding agent libraries.

The notation of Formula (I) 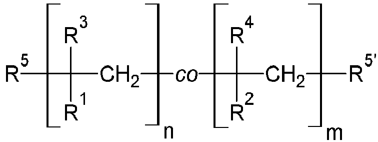 and any other formula herein which discloses a polymer by means of n- and m-numbers of building blocks, is to be understood as follows. The "co", as defined in IUPAC nomenclature, means that the monomers are randomly distributed within the polymer, i.e. there is an unspecified sequence of monomers in the polymer. Optionally, the distribution of the monomers can be alternating or statistical. R⁵ and R^{5'} on either end (alpha and omega end) of the polymer can be a chemical entity or group resulting from the reaction of a polymerization initiator, which may be further functionalized by a TBM. Exemplary chemical entities resulting from polymerization reactions include, e.g., groups resulting from a reversible addition-fragmentation chain transfer (RAFT) reagent, an atom transfer radical polymerization (ATRP) reagent or free-radical polymerization (FRP) reagent. These reagents and the products of the reagents that are formed as groups on the polymer ends are well known in the art. For example, RAFT reagents are commercially available trithiocarbonates, dithiocarbamates, dithiobenzoates, switchable RAFT reagents or macro-RAFT reagents, e.g. as available from Sigma Aldrich Canada Co. or Merck KGaA, Darmstadt, Germany. It is within the skilled person's general chemical knowledge what the structure of R⁵ and/or R^{5'} will be when choosing a specific polymerization initiator. Alternatively, R⁵ and/or R^{5'} may be a chemical entity resulting from a polymerization reaction as described herein which entity is further chemically modified (e.g. reacted with a TBM) to be a target binding moiety, or R⁵ and/or R^{5'} may be a target binding moiety in the absence of a chemical entity resulting from a polymerization. The term "a TBM attached to or reacted with a group resulting from a polymerization initiator" means that a TBM is attached to the group by means of a chemical reaction to form, e.g. a covalent or ionic, bond with the chemical entity resulting from a polymerization. This attachment may result in the direct attachment of the TBM to the polymer under loss of the chemical entity resulting from a polymerization or it may result in the chemical modification of the chemical entity, e.g. partial loss of chemical entity.

In any case, the target binding moiety is optionally selected from the group consisting of azide, alkyne, carboxylate, amine, thiol, hydroxyl, aldehyde, cyanate, sulfonyl, tosyl, tresyl, epoxide, carbonate, halide, anhydride, carbamate, imidazole, azlactone, triazine, maleimide, aziridine, peroxide, acyl, anthraquinone, diazo, diazirine, psoralen, NHS ester, imido ester, (C₂₋₁₀)alkenyl or alkyl groups, DBCO, cytosine, guanidine, streptavidin, avidin, biotin, an aptamer, a peptide, a protein, an oligonucleotide, a nucleic acid, a carbohydrate, a dye, a cell, an antibody, an organic and inorganic nanoparticle, hydrophilic uncharged oligopolymers, positively and negatively charged oligopolymers.

The polymer described herein for all aspects and embodiments may be crosslinked by any suitable crosslinker. A crosslinker is a chemical agent that crosslinks the polymer, i.e. binds together two or more polymer chains, by attaching via at least two moieties of the same crosslinker to two or more polymer chains. The crosslinking can be reversible or irreversible and it can be based on a chemical linkage or on non-covalent interactions between the crosslinker and the polymer. Suitable crosslinkers for polymers are known to the skilled person and include but are not limited to, e.g. bismethacrylates, bismethacrylamides, and glutharaldeyde. Examples of non-covalent crosslinkers include, e.g., streptavidin that is able to bind with biotin-conjugated polymers or nanoparticles/proteins/compounds decorated with multiple antibodies or targeting agents.

The target binding moiety (TBM), as used herein for all aspects and embodiments, includes any suitable chemical or biological moiety that can bind a desired target, e.g. a compound to be taken up by the droplets, reversibly or irreversibly, covalently or non-covalently. Optionally, the target binding moiety is chosen such that it binds only to the desired product when in use together as part of the liquid droplets described herein. Examples for reactive groups or moieties for bioconjugations are known in the art (see, e.g., Greg T. Hermanson, Chapter 3 - The Reactions of Bioconjugation, Bioconjugate Techniques (Third Edition), 2013, Pages 229-258). For example, the TBM can be 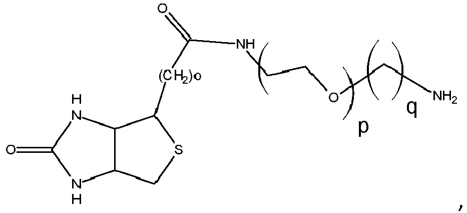 wherein o in (CH₂)o is an integer from 1 to 10, optionally 2 to 5, p is an integer from 1 to 5, optionally 2, and q is an integer from 1 to 5, and the TBM is connected via the amine to the polymer directly or to the entity resulting from polymerization described herein. Optionally, the TBM can be 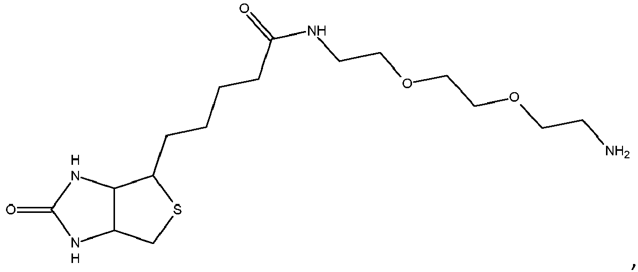 attached via the amine to the polymer described herein, optionally to R⁵ or R^{5'} being 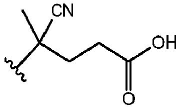 and from an amide bond.

The term DBCO, as used herein, refers to dibenzocyclooctyne, which is useful, e.g., in strain-promoted copper-free azide-alkyne cycloaddition reactions.

An antibody, as used herein for all aspects and embodiments, includes functional fragments and functional derivatives thereof or antibody-like binding proteins that bind a desired target, e.g. a compound to be taken up by the droplets. As used herein, the term antibody is meant to include whole antibodies, functional fragments and functional derivatives thereof that specifically bind a desired target. These are routinely available by hybridoma technology (Kohler and Milstein, Nature 256, 495-497, 1975), antibody phage display (Winter et al., (1994) Annu. Rev. Immunol. 12, 433-455), ribosome display (Schaffitzel et al., (1999) J. Immunol. Methods, 231, 119-135) and iterative colony filter screening (Giovannoni et al., (2001) Nucleic Acids Res. 29, E27) once the desired target is available. Typical proteases for fragmenting antibodies into functional products are well-known. Other fragmentation techniques can be used as well as long as the resulting fragment has a specific high affinity and, preferably a dissociation constant in the micromolar to picomolar range. A very convenient antibody fragment for targeting applications is the single-chain Fv fragment, in which a variable heavy and a variable light domain are joined together by a polypeptide linker. Other antibody fragments for target binding include Fab fragments, Fab₂ fragments, miniantibodies (also called small immune proteins), tandem scFv-scFv fusions as well as scFv fusions with suitable domains (e.g. with the Fc portion of an immuneglobulin). For a review on certain antibody formats, see Holliger P, Hudson PJ.; Engineered antibody fragments and the rise of single domains. Nat Biotechnol. 2005 Sep., 23(9):1126-36). The term "functional derivative" of an antibody for use in the present invention is meant to include any antibody or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative has substantially the same binding affinity as to its original antigen and, optionally, has a dissociation constant in the micro-, nano- or picomolar range. The antibody, fragment or functional derivative thereof for use in the present invention can be, for example, one that is selected from the group consisting of polyclonal antibodies, monoclonal antibodies, chimeric antibodies, humanized antibodies, CDR-grafted antibodies, Fv-fragments, Fab-fragments and Fab₂-fragments and antibody-like binding proteins, e.g. affilines, anticalines and aptamers, any protein-ligand binding pair and the fragments of the both. For a review of antibody-like binding proteins see Binz et al. on engineering binding proteins from non-immunoglobulin domains in Nature Biotechnology, Vol. 23, No. 10, October 2005, 12571268. The term "aptamer" describes nucleic acids that bind to a polypeptide with high affinity. Aptamers can be isolated from a large pool of different single-stranded RNA molecules by selection methods such as SELEX (see, e.g., Jayasena, Clin. Chem., 45, p. 1628 - 1650, (1999); Klug and Famulok, M. Mol. Biol. Rep., 20, p. 97 - 107 (1994); US 5,582,981). Aptamers can also be synthesized and selected in their mirror form, for example, as the L-ribonucleotide (Nolte et al., (1996) Nat. Biotechnol., 14, pp.1116 - 1119; Klussmann et al., (1996) Nat. Biotechnol. 14, p. 1112 - 1115). Forms isolated in this way have the advantage that they are not degraded by naturally occurring ribonucleases and, therefore, have a greater stability. Another antibody-like binding protein and alternative to classical antibodies are the so-called "protein scaffolds", for example, anticalines, that are based on lipocaline (Beste et al., (1999) Proc. Natl. Acad. Sci. USA, 96, p. 1898 - 1903). The natural ligand binding sites of lipocalines, for example, of the retinol-binding protein or bilin-binding protein, can be changed, for example, by employing a "combinatorial protein design" approach, and in such a way that they bind selected haptens (Skerra, (2000) Biochem. Biophys. Acta, 1482, pp. 337 - 350). For other protein scaffolds it is also known that they are alternatives for antibodies (Skerra, (2000) J. Mol. Recognit, 13, pp. 167 - 287; Hey, (2005) Trends in Biotechnology, 23, pp. 514-522). In summary, the term functional antibody derivative is meant to include the above protein-derived alternatives for antibodies, i.e. antibody-like binding proteins, e.g. affilines, anticalines and aptamers that specifically recognize a desired target, compound, polypeptide, fragment or derivative thereof.

Examples for dyes, as used herein for all aspects and embodiments include, e.g., ATTO-488, ATTO390, ATTO647N, ATTO565, ALEXA488, Rhodamine B, Fluorescein, Amplex Red, fluorescamine, aromatic dialdehyde naphthalene-2,3-dicarboxaldehyde (NDA), or fluoraldehyde.

Examples for cells, as used herein for all aspects and embodiments include, e.g., stem cells, dendritic cells, microglia, beta cells, T cells, lymphocytes, astrocytes, E. coli, osteoblasts, or HEK cells.

Examples for organic and inorganic nanoparticles, as used herein for all aspects and embodiments include, e.g., magnetic nanoparticles, quantum dots, metal-organic framework (MOF) nanoparticles, or biodegradable nanoparticles, e.g. poly(caprolactone) (PCL), poly(lactic acid) (PLA), or poly(lactic-co-glycolic acid) (PLGA) based-nanoparticles.

Examples for hydrophilic uncharged oligopolymers, positively and negatively charged oligopolymers, as used herein for all aspects and embodiments, include, e.g., poly(methacrylic acid sodium salt), poly(2-dimethylamino)ethyl methacrylate) methyl chloride quaternary salt, chitosan, heparin, sodium alginate, poly(ethylene glycol), and poly(N-(2-hydroxypropyl)methacrylamide).

The controlled uptake of compounds from outside the droplets into the droplets for storage, separation and/or reaction of compounds inside the droplets means that the droplets selectively take up compounds from the environment, i.e. exterior of the droplets, into the droplets and store these compounds inside the droplets without significant leakage of the same compounds. Once stored, the compounds may undergo chemical or biological reactions within the droplets and subsequently may or may not be released from the droplets, e.g. based on their altered chemical composition or properties. The separation of the compounds inside the droplets can refer to the separation of compounds originating from outside the droplets and it can refer to the separation of different compounds located within the droplets, e.g. based on different chemical properties of the compounds that interact with different TBMs or R¹/R² structures of the polymer of the droplets, e.g. leading to compartments or sub-compartments within the same droplet for compound separation as described further below.

The process described herein for uptake of compounds from outside the droplets into the droplets is not to be confused with the loading of, e.g. solid, vesicles in the context of drug delivery. The droplets for use in the present invention are not used or assembled together with high concentrations of a compound in order to load the compound into the droplets. Rather, the droplets are used in environments where the compounds should be taken up by the already existing droplets, e.g. at relatively low concentrations of the compounds, into the droplets and subsequently are not released from the droplets without alteration of the compounds or the droplets.

The compounds that are taken up by the droplets described herein can be any chemical or biological compound, including, e.g. small molecules, drugs, antibodies, antigens, RNA, nucleic acids, viruses, carbohydrates, membrane-bound vesicles, contaminants, DNA, exosomes, extracellular vesicles, cells, proteins, peptides, biomolecules, enzymes, and ab42.

As used herein for all aspects and embodiments, a "substituent" or "residue" or **"R",** refers to a molecular moiety that is covalently bound to an atom within a molecule of interest. For example, a "substituent", **"R"** or "residue" may be a moiety such as a halogen, alkyl group, haloalkyl group or any other substituent described herein that is covalently bonded to an atom, optionally a carbon, oxygen or nitrogen atom, that forms part of a molecule of interest.

The group **X** used herein for all aspects and embodiments is selected from the group consisting of -C(=O)-O-, -C(=O)-NH-, aliphatic and aromatic carbo- or heterocycle. In any aspect disclosed herein, the group "-C(=O)-O-" or "-C(=O)-NH-" is annotated such that the attachment point (indicated by the waved line) of the moiety comprising said group is at the beginning of either -C(=O)-O- or -C(=O)-NH-. In other words and as a representative example for all aspects and embodiments, if **X** is selected to be -C(=O)-O- or -C(=O)-NH- in a residue of the following formula: 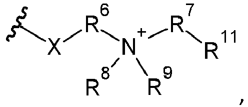 the following structures result: 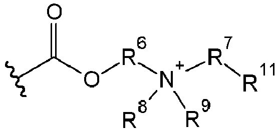 or

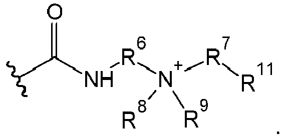

The term "non-substituted" as used herein shall mean substituted only with hydrogen. The term "substituted" as used herein, means that any one or more hydrogens on the designated atom or group is replaced, independently, with an atom different from hydrogen, optionally by a halogen, optionally by fluorine, a thiol, a carboxyl, a cyano, a nitro, an alkyl (optionally C₁-C₁₀), aryl (optionally phenyl, benzyl or benzoyl), an alkoxy group, a sulfonyl group, by a tertiary or quaternary amine or by a selection from the indicated substituents, provided that the designated atom's normal valence is not exceeded, and that the substitution results in a stable compound, i.e., a compound that can be isolated and characterized using conventional means.

The term "heteroatom" as used herein shall be understood to mean atoms other than carbon and hydrogen such as, e.g., O, N, S and P.

In the context of the present invention it is understood that antecedent terms such as "linear or branched", "substituted or non-substituted" indicate that each one of the subsequent terms is to be interpreted as being modified by said antecedent term. For example, the scope of the term "linear or branched, substituted or non-substituted alkyl, alkenyl, alkynyl, carbocycle" encompasses linear or branched, substituted or non-substituted alkyl; linear or branched, substituted or non-substituted alkenyl; linear or branched, substituted or non-substituted alkynyl; linear or branched, substituted or non-substituted alkylidene; and linear or branched, substituted or non-substituted carbocycle. For example, the term "(C₂₋₁₀) alkenyl, alkynyl or alkylidene" indicates the group of compounds having 2 to 10 carbons and alkenyl, alkynyl or alkylidene functionality.

The expression "alkyl" refers to a saturated, straight-chain or branched hydrocarbon group that contains the number of carbon items indicated, e.g. linear or branched "(C₁₋₁₀)alkyl" denotes a hydrocarbon residue containing from 1 to 10 carbon atoms, e.g. a methyl, ethyl, propyl, iso-propyl, n-butyl, iso-butyl, sec-butyl, tert-butyl, n-pentyl, iso-pentyl, n-hexyl, 2,2-dimethylbutyl, etc.

The expression "alkenyl" refers to an at least partially unsaturated, substituted or non-substituted straight-chain or branched hydrocarbon group that contains the number of carbon atoms indicated, e.g. "(C₂₋₁₀)alkenyl" denotes a hydrocarbon residue containing from 2 to 10 carbon atoms, for example an ethenyl (vinyl), propenyl (allyl), iso-propenyl, butenyl, isoprenyl or hex-2-enyl group, or, for example, a hydrocarbon group comprising a methylene chain interrupted by one double bond as, for example, found in monounsaturated fatty acids or a hydrocarbon group comprising methylene-interrupted polyenes, e.g. hydrocarbon groups comprising two or more of the following structural unit -[CH=CH-CH₂]-, as, for example, found in polyunsaturated fatty acids. Alkenyl groups have one or more, e.g. 1, 2, 3, 4, 5, or 6 double bond(s).

The expression "alkyne" refers to at least partially unsaturated, substituted or non-substituted straight-chain or branched hydrocarbon groups that may contain, e.g. from 2 to 10 carbon atoms, for example an ethinyl, propinyl, butinyl, acetylenyl, or propargyl group. Optionally, alkine groups have one or two (e.g. one) triple bond(s).

Furthermore, the terms "alkyl", "alkenyl" and "alkyne" also refer to groups in which one or more hydrogen atom(s) have been replaced, e.g. by a halogen atom, optionally F, CI or Br, such as, for example, a 2,2,2-trichloroethyl, tribromoethyl or a trifluoromethyl group.

The term "heterocycle" refers to a stable substituted or non-substituted, aromatic or nonaromatic, optionally 3 to 10 membered, optionally 3 - 6 membered, optionally 5 or 6 membered, monocyclic, heteroatom-containing cycle. Each heterocycle consists of carbon atoms and one or more, optionally 1 to 4, optionally 1 to 3 heteroatoms optionally chosen from nitrogen, oxygen and sulphur. A heterocycle may contain the number of carbon atoms in addition to the non-carbon atoms as indicated: a "(C₃₋₆)heterocycle" is meant to have 3 to 6 carbon atoms in addition to a given number of heteroatoms.

Exemplary heterocycles include, but are not limited to pyridine, piperidine, pyrrole, pyrimidine, pyrazole, imidazole, imidazole, pyrazine, isoxazole or oxazole.

The expressions "alkyl ether" refers to a saturated or non-saturated, straight-chain or branched hydrocarbon group that contains the number of carbon items indicated. For example, "(C₂₋₅)alkyl ether" denotes a hydrocarbon residue containing from 2 to 5 carbon atoms, and any suitable number of oxygen atoms that will result in an ether structure. Alkyl ether groups as used herein, shall be understood to mean any linear or branched, substituted or non-substituted alkyl chain comprising an oxygen atom either as an ether motif, i.e. an oxygen bound by two carbons. Exemplary alkyl ethers are polyethylene glycol (PEG) chains. The term polyethylene glycol as used herein refers to a chain of substituted or non-substituted ethylene oxide monomers. The ether residue can be attached to the Formula provided in the present invention either via the carbon atom or via the oxygen atom of the ether residue.

If more than one residue **R** is attached to a given atom of a formula described herein, one residue **R** can be absent if the attachment of the other **R** leads to a full valency of the atom. For example, **R⁸** or **R⁹** is absent if **R⁸** and **R⁶, R⁹** and **R⁶** or **R⁸** and **R⁷,** or **R⁹** and **R⁷** together form an aromatic ring.

As used herein, a wording defining the limits of a range of length such as, e. g., "from 1 to 5" or "(C₁₋₅)" means any integer from 1 to 5, i.e. 1, 2, 3, 4 and 5. In other words, any range defined by two integers explicitly mentioned is meant to comprise and disclose any integer defining said limits and any integer comprised in said range.

The scope of the present invention includes those analogs of the compounds as described above and in the claims that feature the exchange of one or more carbon-bonded hydrogens, optionally one or more aromatic carbon-bonded hydrogens, with halogen atoms such as F, CI, or Br, optionally F. The exchange of one or more of the carbon-bonded hydrogens, e.g. by fluorine, can be done, e.g., for reasons of metabolic stability and/or pharmacokinetic and physicochemical properties.

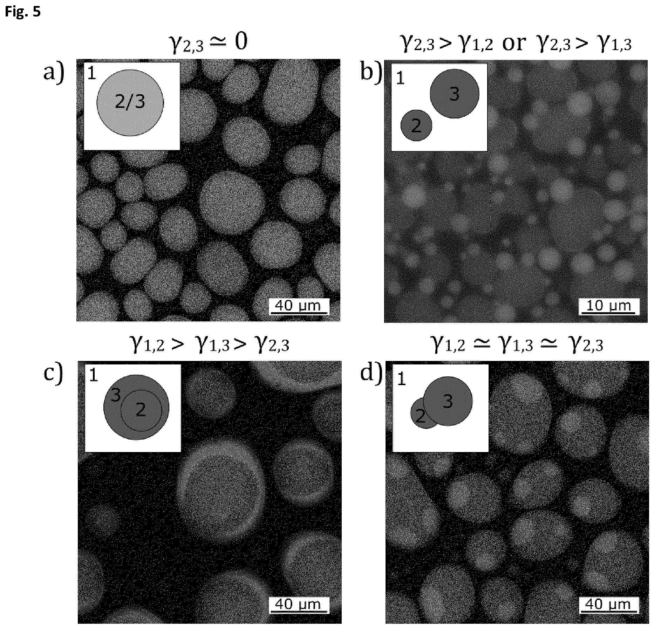

In an embodiment, the droplets as such or in the context of the use of the present invention are multiphase immiscible droplets, wherein compartments of at least two different droplets can be fully immiscible or form sub-compartments. The compartments are to be understood as the "interior" of the droplets. The sub-compartments of at least two different droplets may have a core-shell morphology and/or feature partial wetting, e.g. as shown in Fig. 5. The full or partial immiscibility of the at least two droplets described herein for any aspect is based on the different chemical nature of the polymer of the at least two droplets.

For example, when two different polymers described herein are mixed, these two form distinct immiscible droplets that are composed only of one single type of polymer (i.e. if polymer A is mixed with polymer B, two types of droplets are obtained, wherein one type of droplet is composed only of polymer A and the other one is composed only of polymer B). These immiscible droplets can be fully separated, partially wetting or feature a core shell morphology.

As an example, the above properties of the droplets to separate based on the nature of the polymer they are made of can be used to detect two or more different compounds (e.g. biomarkers) at the same time from the same complex mixture. At the end, multiple immiscible droplets each containing a specific compound or biomarker can be obtained and detected simultaneously from the same complex sample mixture (e.g. a blood sample).

For example, fine-tuning the different responsiveness of the droplets may be used to recover two recruited compounds. As an example, the droplets can be sequentially disassembled and the compounds can be recovered at different pH, temperature or ionic strength.

In an embodiment of all aspects descried herein, R⁵ and/or R^{5'} are selected from 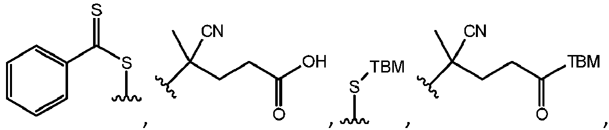 optionally 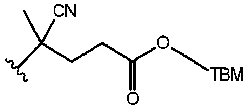 or 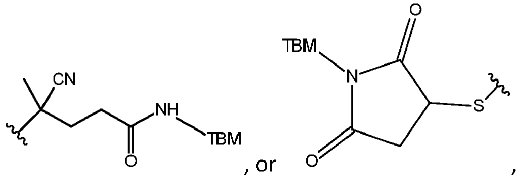 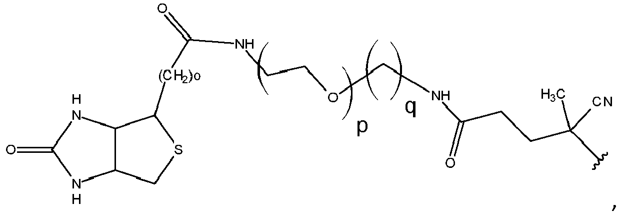 or 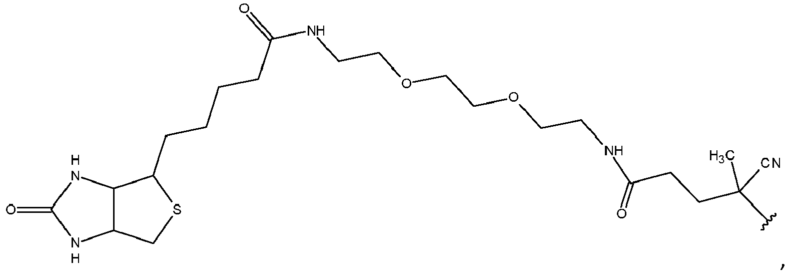 wherein o in (CH₂)o is an integer from 1 to 10, optionally 2 to 5, p is an integer from 1 to 5, optionally 2, and q is an integer from 1 to 5.

In an embodiment, the use of the present invention is one, wherein
**n** is an integer selected from 10 to 250;
**m** is an integer selected from 10 to 250;
**e** is an integer selected from 2 to 3;
**X** is selected from the group consisting of -C(=O)-O-, -C(=O)-NH-, pyridine, piperidine and phenyl;
**R⁶** and **R⁷** are each independently selected from the group consisting of (C₁₋₅)alkyl, optionally methyl, ethyl and propyl;
**R⁸, R⁹** and **R¹⁰** are each independently selected from the group consisting of methyl, 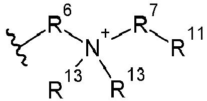 when forming R¹, and 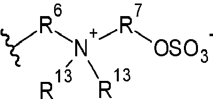 when forming R²; **R¹¹** is -SO₃⁻ or -C(=O)O⁻;
**R¹²** is methyl; and
**R¹³** is selected from the group consisting of methyl, ethyl and propyl.

In an embodiment, the use of the present invention is one, wherein
**R¹** is selected from the group consisting of
(i) 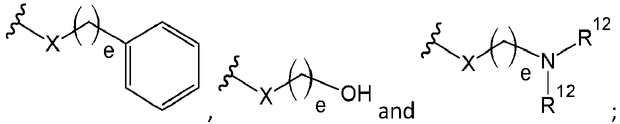
(ii) 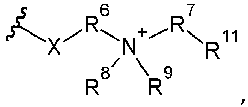 optionally 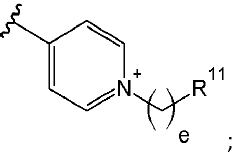
(iii) 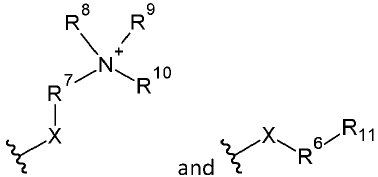 in a ratio of about 1:1 within the polymer;
(iv) 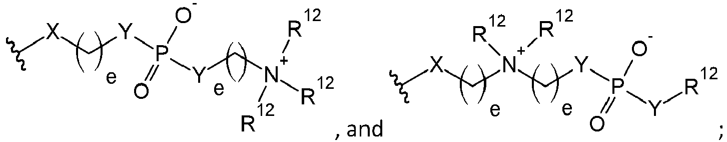 and
(v) 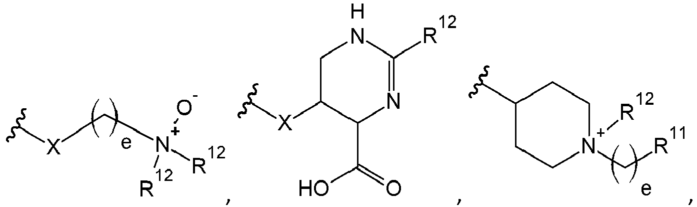 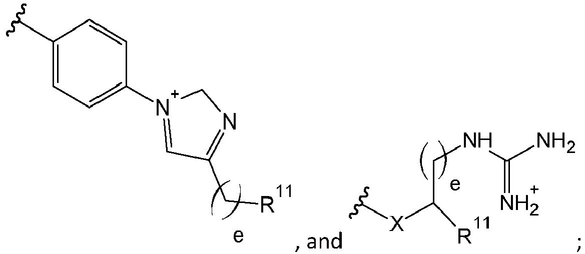 and
   **R²** is

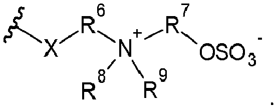

In an embodiment, the use of the present invention is one, wherein
**n** is an integer selected from 10 to 250;
**m** is an integer selected from 10 to 250;
**R⁵** and **R^{5'}** are hydrogen or independently selected from the group consisting of azide, carboxylic acid, amine, (C₂₋₁₀)alkenyl, streptavidin, avidin, biotin, an aptamer, a peptide, a protein, an oligonucleotide, a cell, an antibody, a hydrophilic uncharged oligopolymer, a positively or negatively charged oligopolymer, and an organic or inorganic nanoparticle;
**R¹** is selected from the group consisting of
(i) 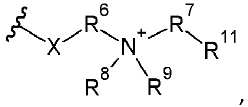
(ii) 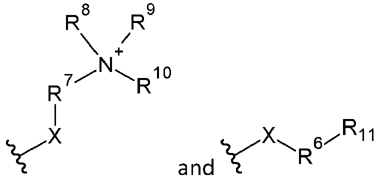 in a ratio of about 1:1 within the polymer;
(iii) 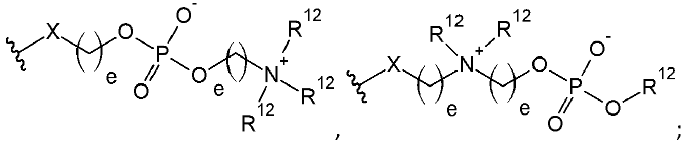 and
(iv) 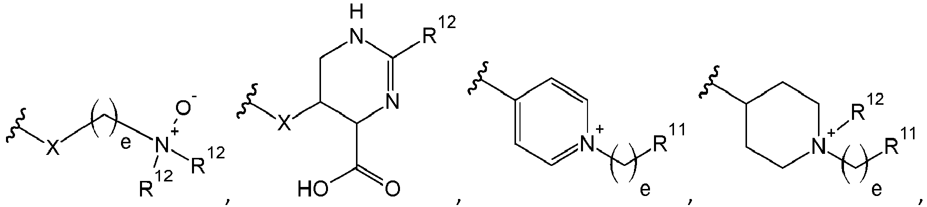 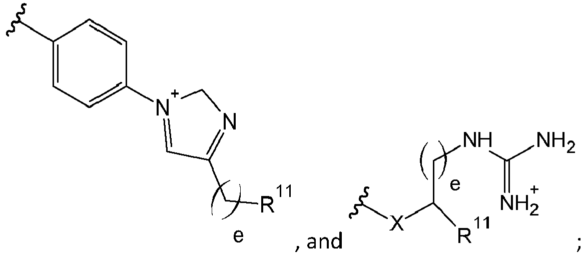 **R²** is 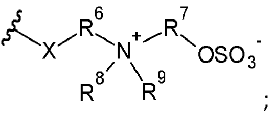 wherein
   **R⁶** and **R⁷** are each independently selected from the group consisting of methyl, ethyl and propyl;
   **R⁸, R⁹, R¹⁰, R¹²** and **R¹³** are each methyl; and
   **R¹¹** is -SO₃⁻ or -C(=O)O⁻.

In an embodiment, the use of the present invention is one, wherein
**R¹** is selected from the group consisting of 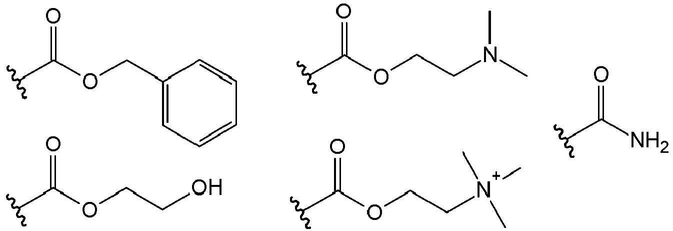 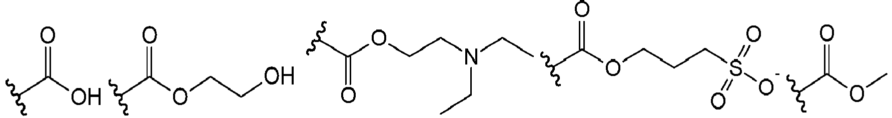 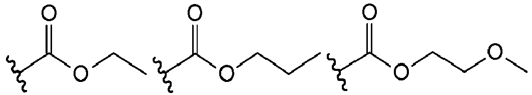 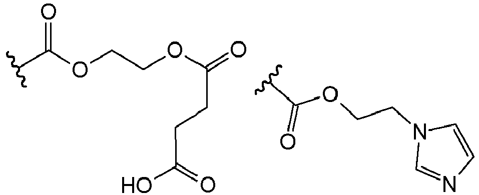 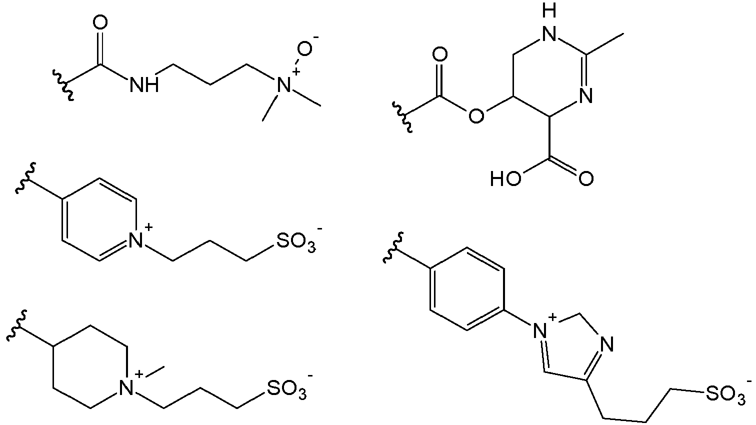 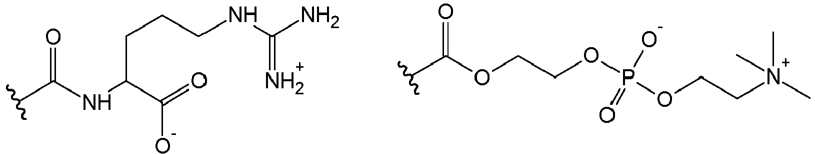 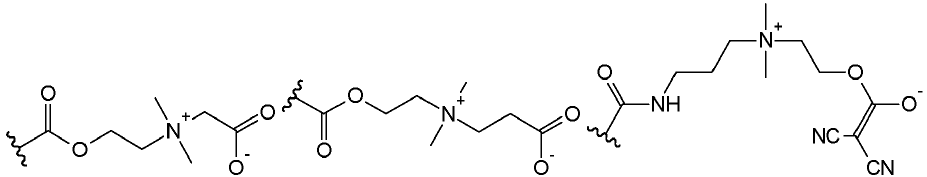 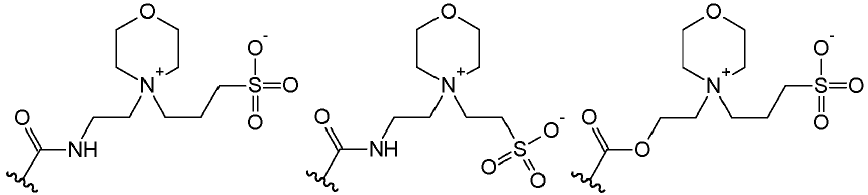 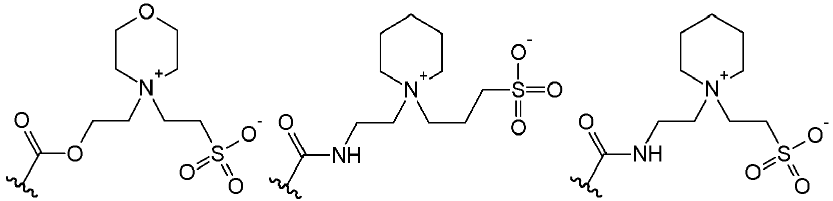 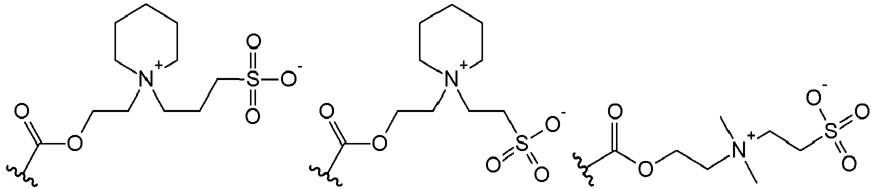 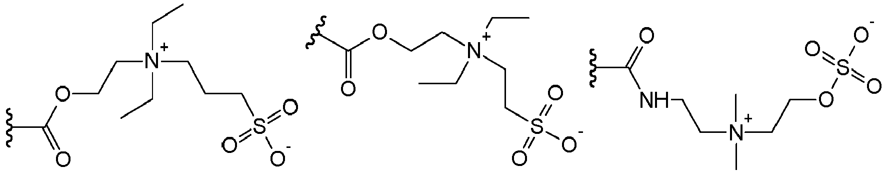 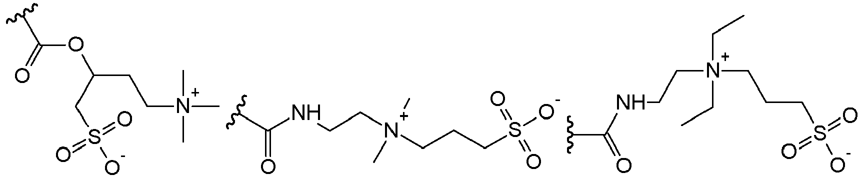 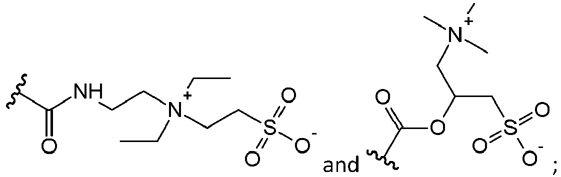 and
**R²** is selected from the group consisting of

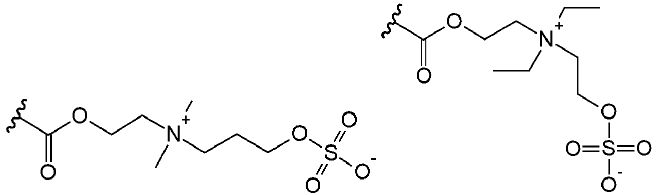

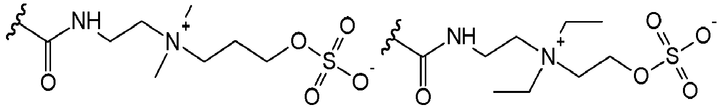

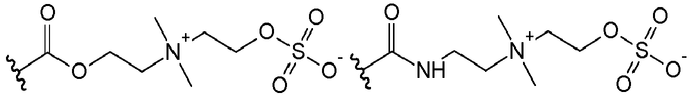

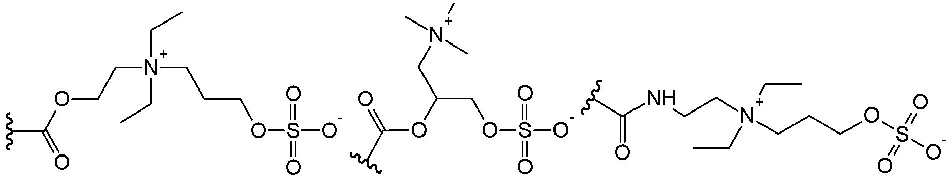

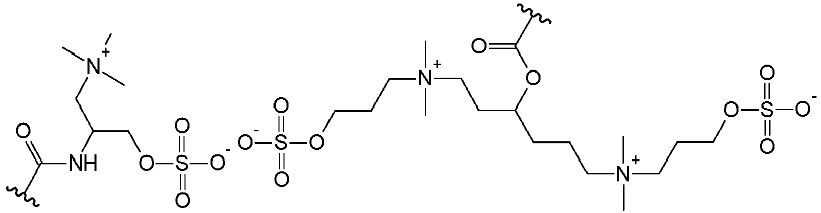

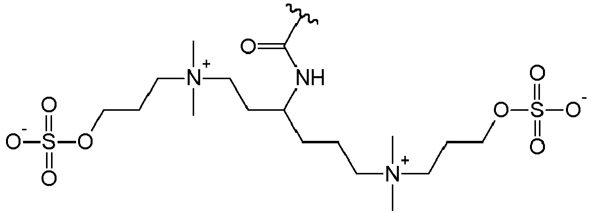

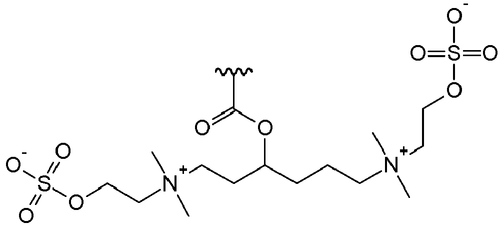

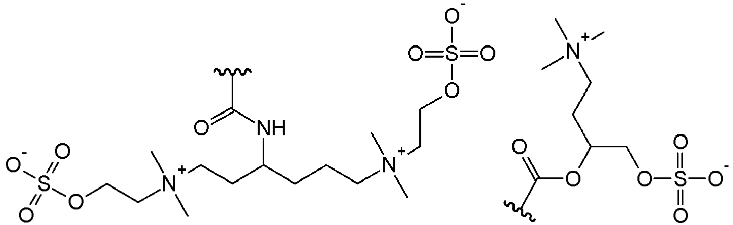

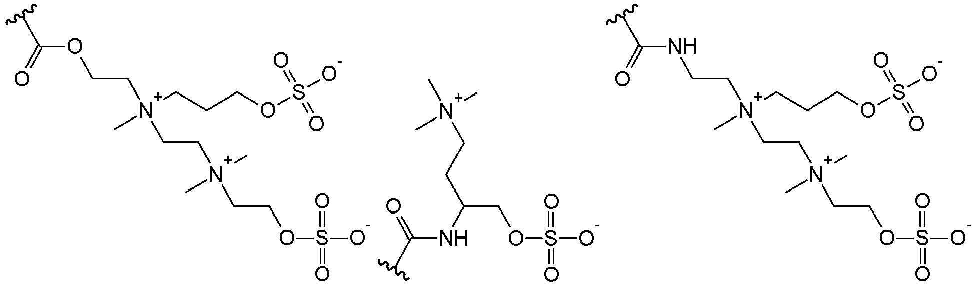

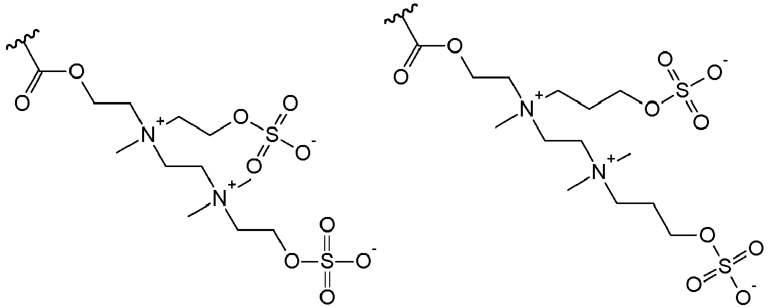

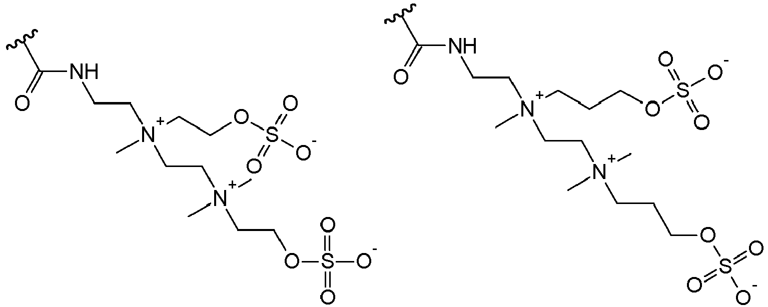

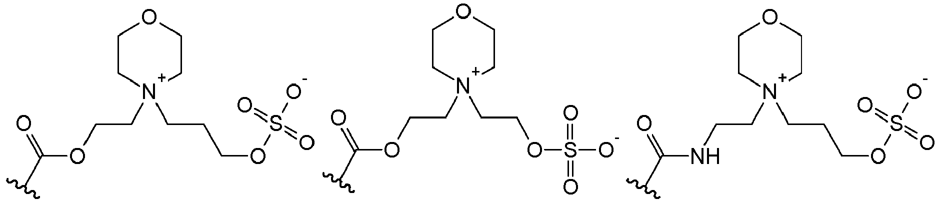

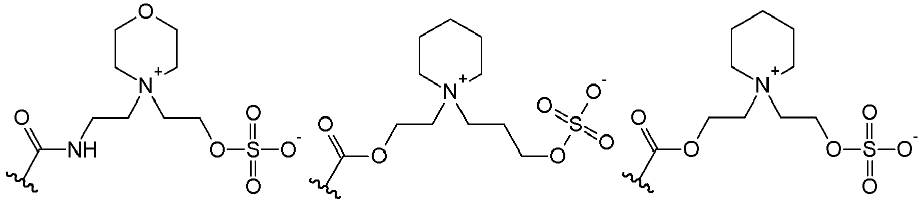

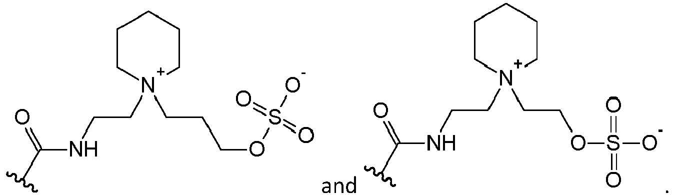

In an embodiment, the use of the present invention is one, wherein the polymer is selected from the group consisting of 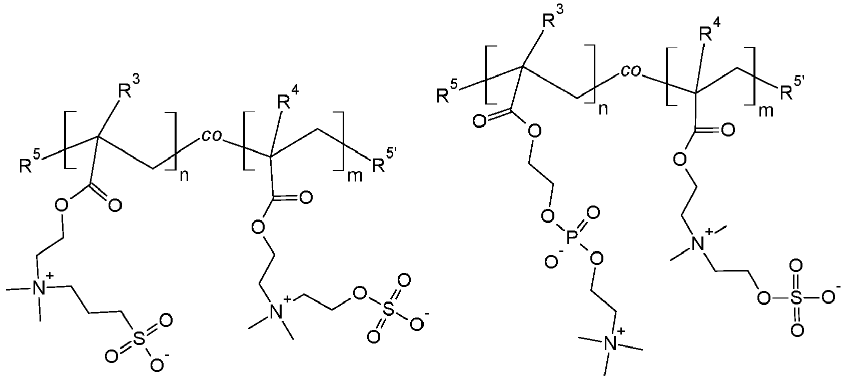 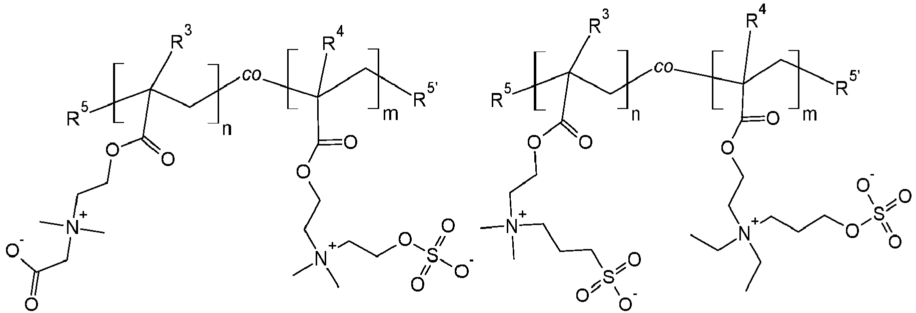 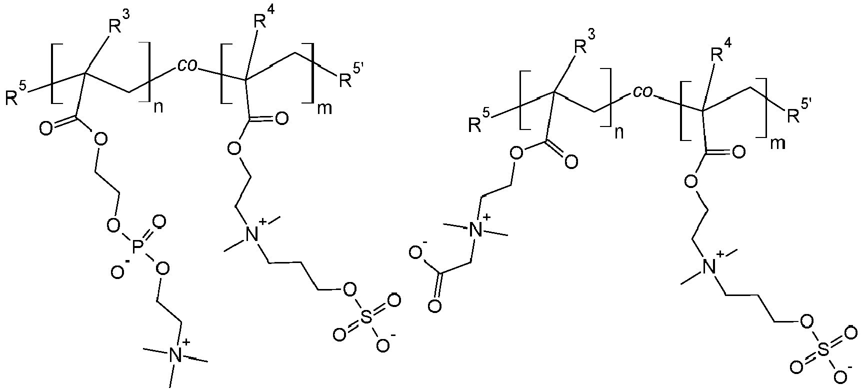 and

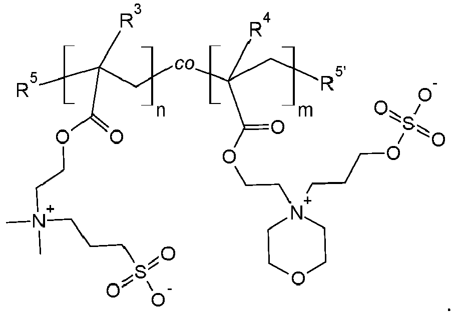

Optionally, R⁵ and R^{5'} of the above-noted polymers are selected from a group resulting from a RAFT polymerization initiator, optionally 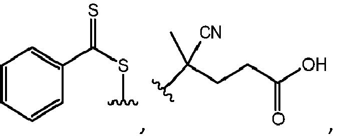 or a target binding moiety (TBM), optionally a TBM attached to or reacted with a group resulting from a polymerization initiator, optionally from a RAFT polymerization initiator, optionally 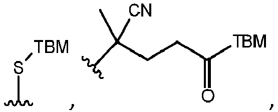 and 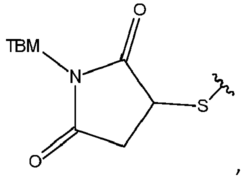 optionally 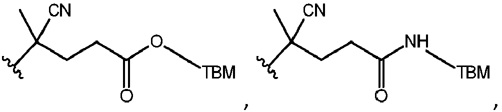 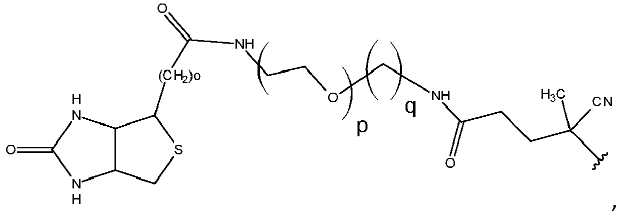 and 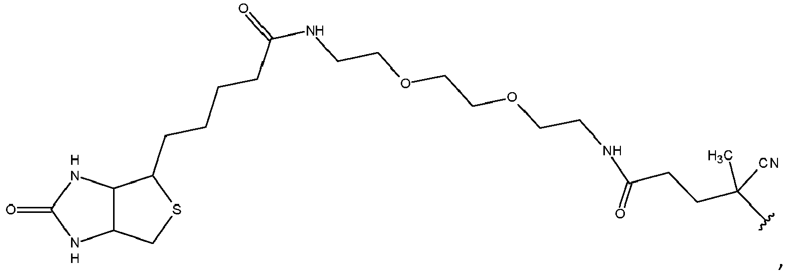 wherein o in (CH₂)o is an integer from 1 to 10, optionally 2 to 5, p is an integer from 1 to 5, optionally 2, and q is an integer from 1 to 5.

The exemplary combinations of R¹ and R² described herein in the context of the use are also meant to be specifically disclosed in all other aspects of the present invention, i.e. in the context of the polymer as such (with the limitations provided below) and in the context of the method.

In an embodiment, the use of the present invention is one, wherein the crosslinker is selected from the group consisting of 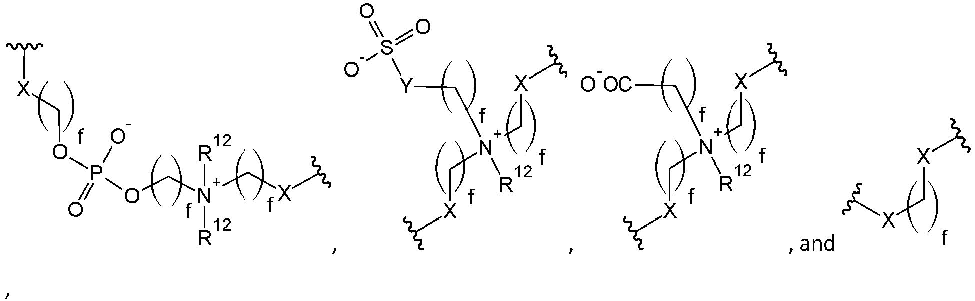 wherein
**f** is an integer selected from 1 to 5, optionally from 2 to 3;
**X** is selected from the group consisting of -C(=O)-O-, -C(=O)-NH-, aliphatic and aromatic carbo- or heterocycle, optionally -C(=O)-O- and -C(=O)-NH-;
**Y** is oxygen or absent; and
**R¹²** is selected from the group consisting of methyl, ethyl and propyl;
optionally the crosslinker is selected from the group consisting of

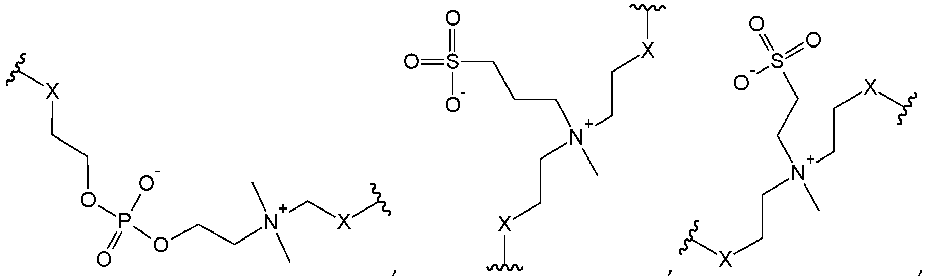

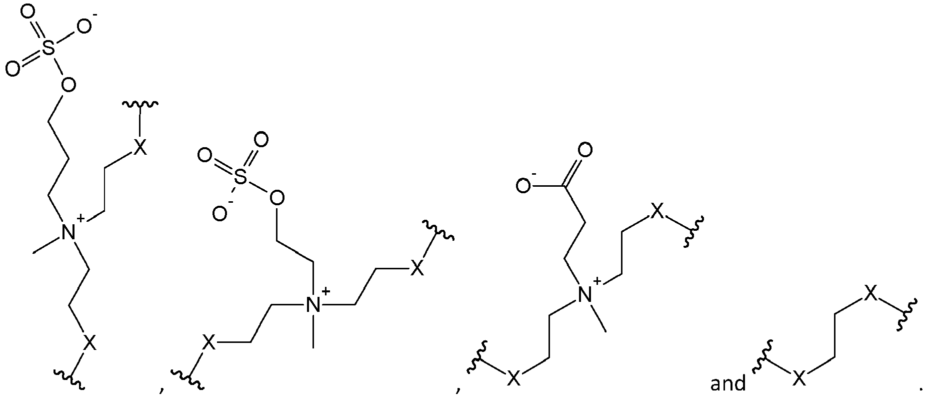

The crosslinker is attached to the backbone of the polymer at the position indicated with the wavy line. The crosslinker can, e.g., crosslink two R¹ or R² positions or an R¹ with an R² position, or an R² with an R¹ position in the polymer backbone.

In an embodiment, the use of the present invention is one, wherein
a. the polymer has a degree of polymerization (n+m) from 5 to 5000, optionally from 50 to 500; and/or
b. the percentage of n in the polymer ([n/(n+m)]^{∗}100) is in the range from 0 to 99.5%, optionally 0 to 70%, optionally 20 to 60%; and/or
c. the dispersity of the polymer is in the range of 1 to 5, optionally 1 to 1.5;
d. the crosslinker molar fraction in the polymer is in the range of 0 to 99%, optionally 0 to 5%; and/or
e. the droplets are responsive to temperature, shear, ionic strength, pH and/or a magnetic field.

The degree of polymerization and the dispersity can be determined by known methods. For example, nuclear magnetic resonance (¹H-NMR) can be used for determining the degree of polymerization and polymer dispersity and molecular weight can be evaluated via gel permeation chromatography.

Responsiveness to temperature, ionic strength, pH, shear and/or a magnetic field means, for example, that the droplets can be disassembled and/or assembled upon change of temperature, ionic strength and/or pH. The morphology of the droplets can be changed, e.g., by applying a magnetic field. Moreover, the surface tension, multiphase miscibility, size, polarity, viscosity, water content, and/or density of the droplets can be controlled, e.g., by changing the properties of the surrounding environment (e.g. temperature, ionic strength, pH, shear, and composition).

In an embodiment, the use of the present invention is one, wherein
a. the compounds for uptake, storage and/or reaction are selected from the group consisting of small molecules, drugs, antibodies, antigens, RNA, nucleic acids, viruses, carbohydrates, membrane-bound vesicles, contaminants, DNA, exosomes, extracellular vesicles, cells, proteins, peptides, biomolecules, enzymes, and ab42; and/or
b. the uptake is affinity- and/or binding-controlled, optionally mediated by interactions of the compound with R⁵ and/or R^{5'} of Formula (I).

In an embodiment, the use of the present invention is one, wherein the droplets are used
(i) for diagnosis of compounds, optionally of biomarkers,
(ii) for isolation for removal and/or enrichment of compounds, optionally for water treatment or cleaning,
(iii) for purification, extraction and/or separation of compounds,
(iv) for detoxification,
(v) for drug screening,
(vi) as cell culture scaffolds, and
(vii) in affinity assays.

The interactions of the compound with R⁵ and/or R^{5'} of Formula (I) as noted above means that the interaction can be with R⁵ and/or R^{5'} being or comprising a TBM as defined above.

In another aspect the present invention is directed to a polymer for forming liquid droplets in an aqueous medium, wherein the polymer is a polymer as defined herein with the proviso that
(i) **R¹** is not 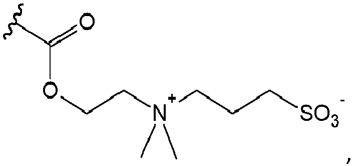 and/or
(ii) **R⁵** and **R^{5'}** are not 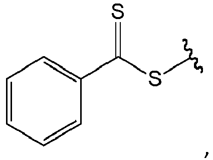 if **R²** is 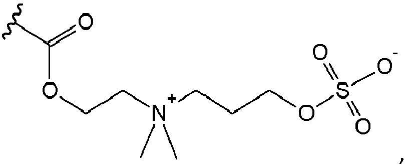 and wherein the droplets take up and/or store compounds in an aqueous medium.

The polymer of the present invention for forming liquid droplets in an aqueous medium can be any polymer described herein in the context of the use and the method as long as the above conditions for R¹, R⁵, R^{5'} and/or R² are met.

All properties and parameters described herein for the polymer in the context of the use can optionally also apply to the polymer for forming liquid droplets in an aqueous medium and to the corresponding method for production, e.g. parameters and properties described herein directed to the degree of polymerization, the percentage of n in the polymer, the dispersity of the polymer, molar fraction of crosslinker and/or the factors to which the polymers are responsive to.

In an embodiment, the polymer of the present invention is one, wherein **R¹** is selected from the group consisting of
(i) -X-hydrogen, -X-methyl, -X-ethyl and -X-propyl; 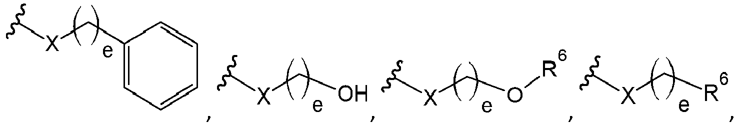
(ii) 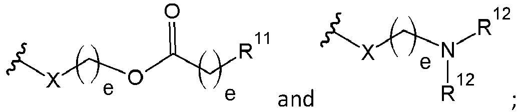
(iii) 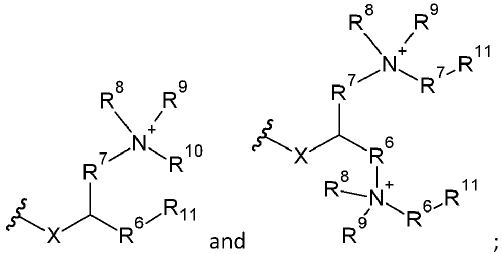
(iv) 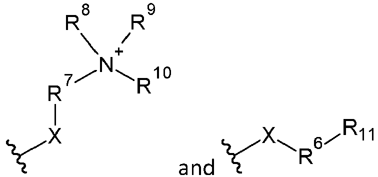 in a ratio of about 1:1 within the polymer;
(v) 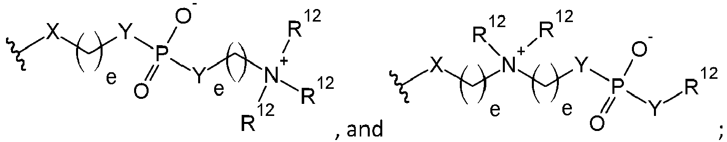 and
(vi)

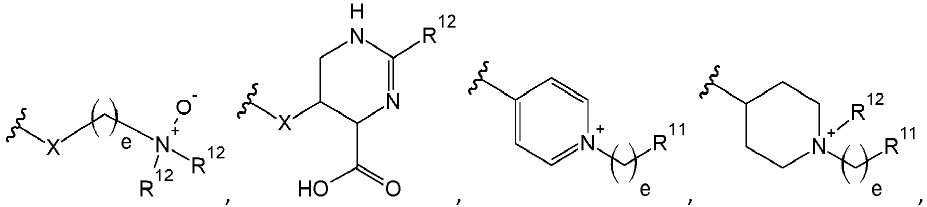

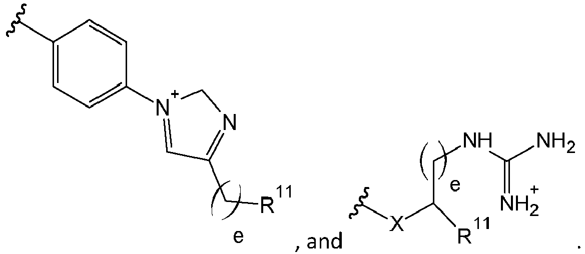

In an embodiment, the polymer of the present invention is one, wherein
**R¹** is selected from the group consisting of 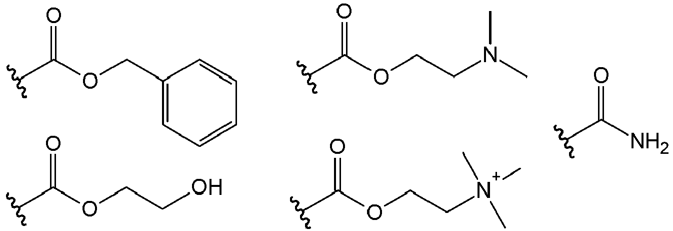 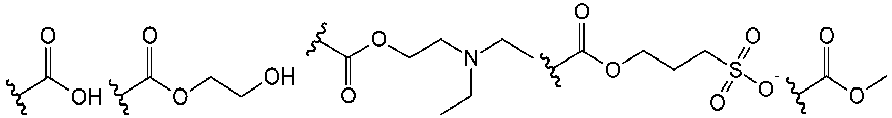 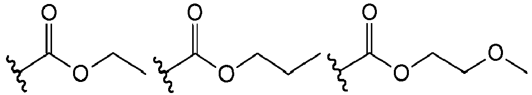 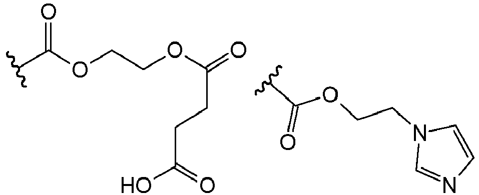 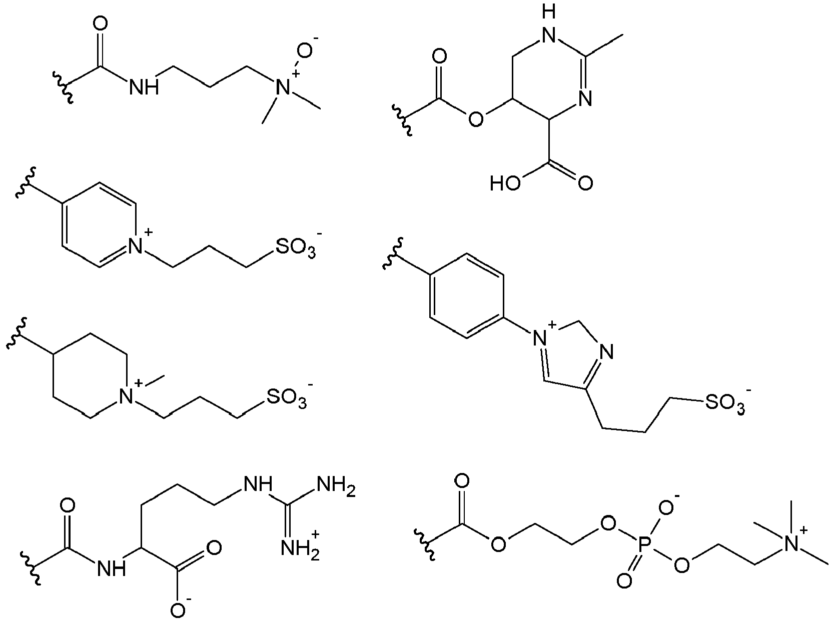 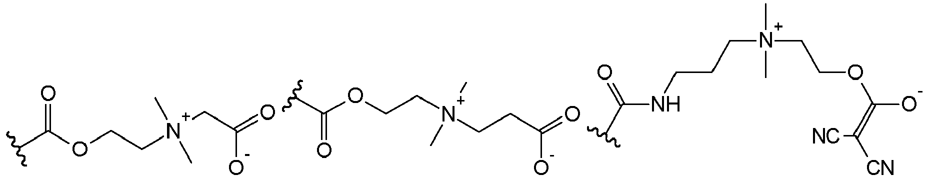 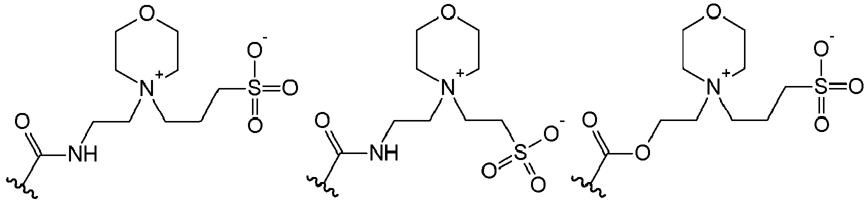 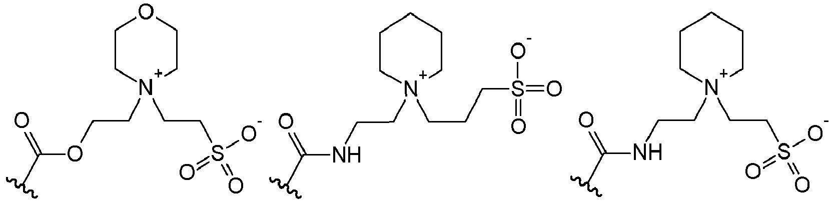 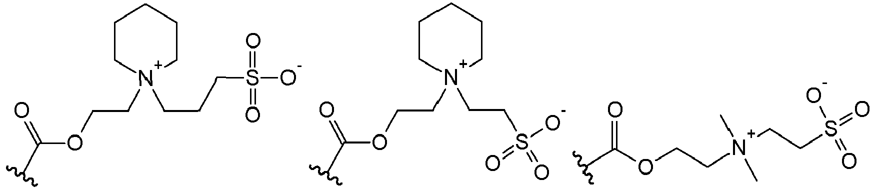 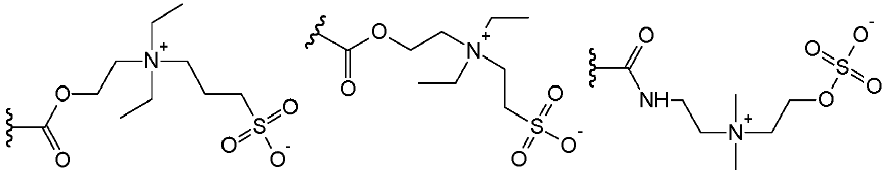 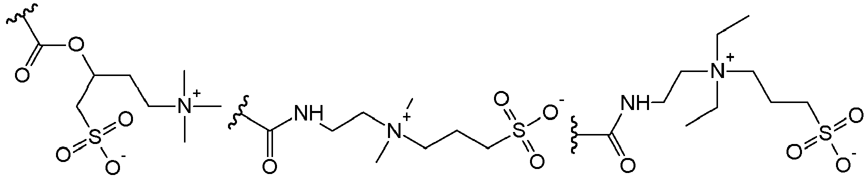 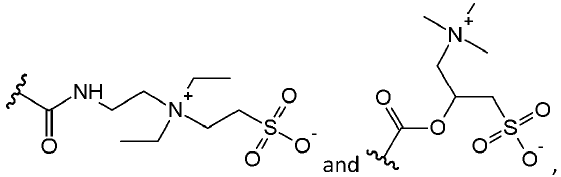 and/or
**R²** is selected from the group consisting of

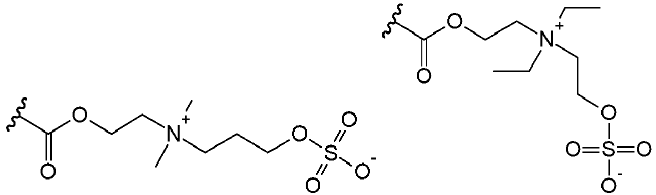

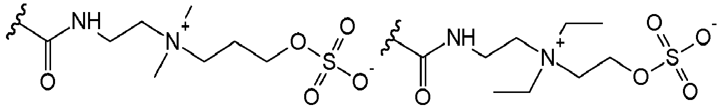

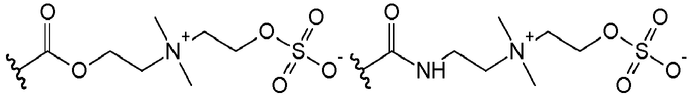

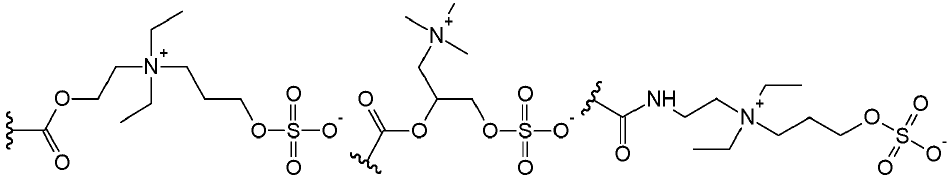

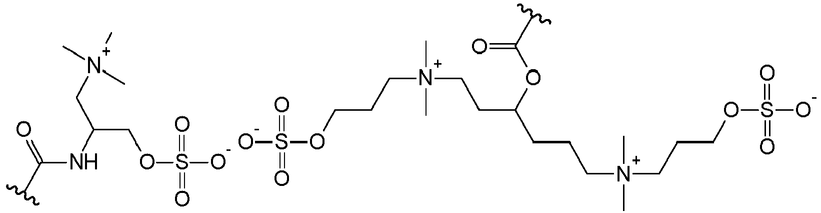

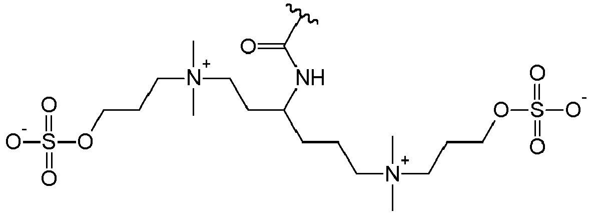

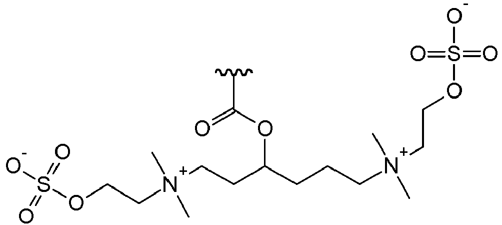

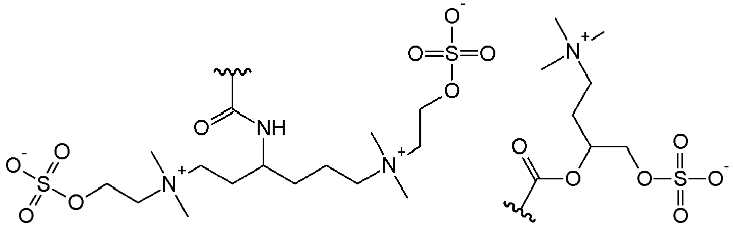

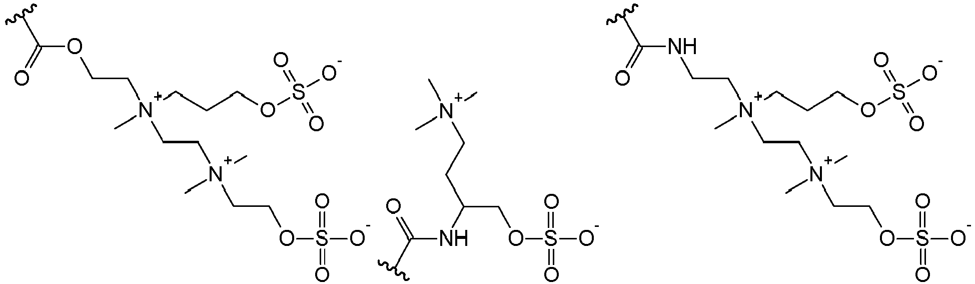

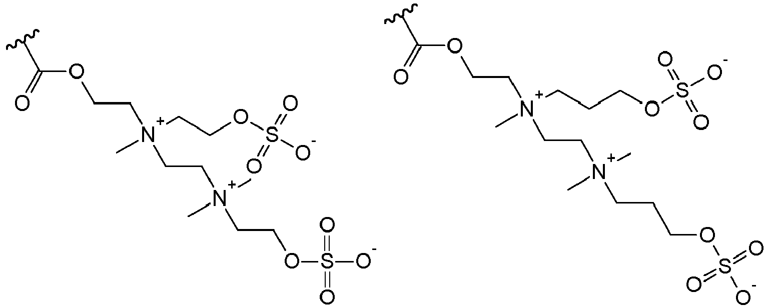

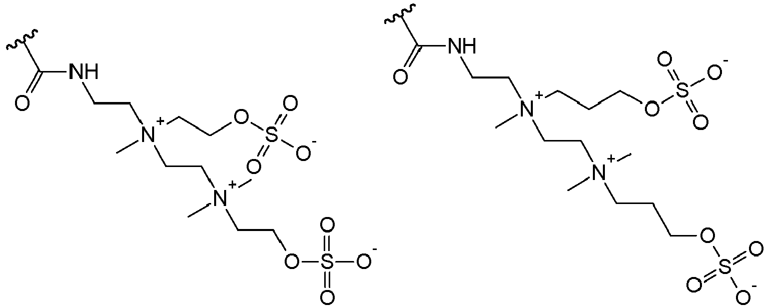

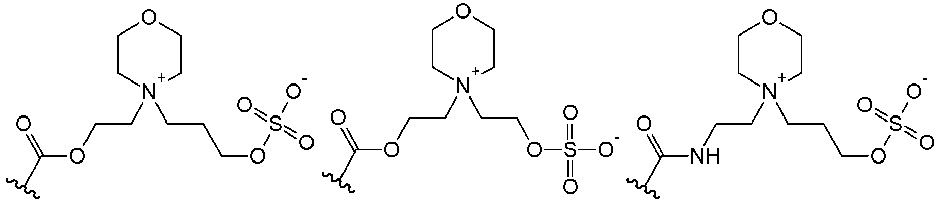

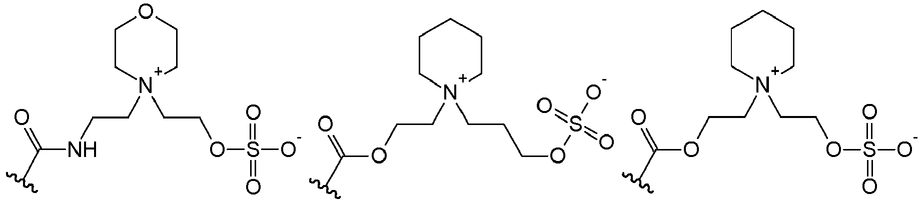

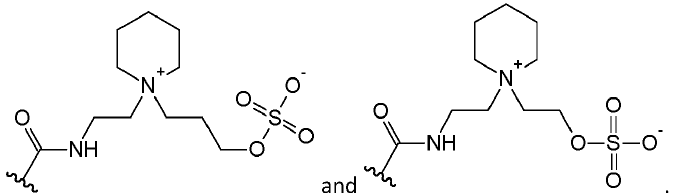

In another aspect, the present invention is directed to a method for producing a polymer for forming liquid droplets in an aqueous medium as described herein comprising the following steps:
(a) providing monomers selected from the group consisting of methyl methacrylate, ethyl methacrylate, propyl methacrylate, butyl methacrylate, hydroxyethyl methacrylate (HEMA), methacrylamide (Mam), benzyl methacrylate (BeMA), 2-dimethylaminoethyl methacrylate methyl chloride (MADQUAT) and 2-dimethylaminoethyl methacrylate (DMAEMA), 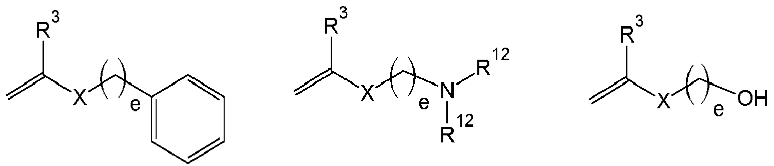 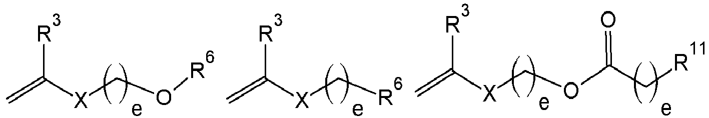 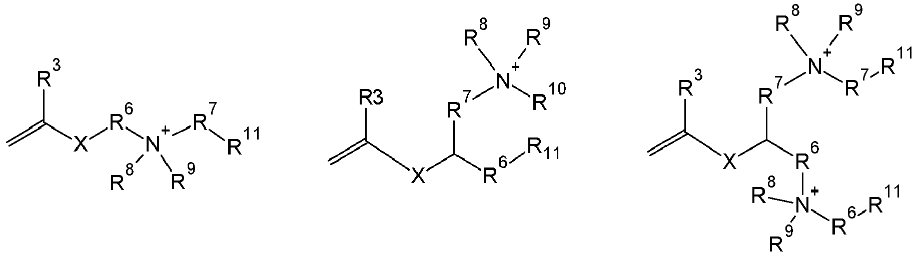 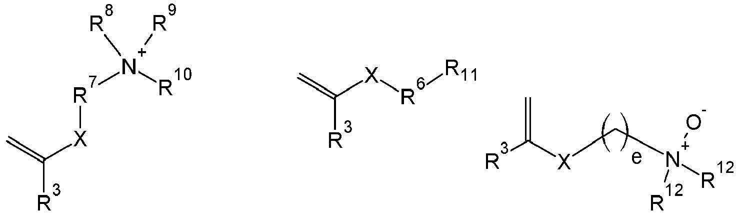 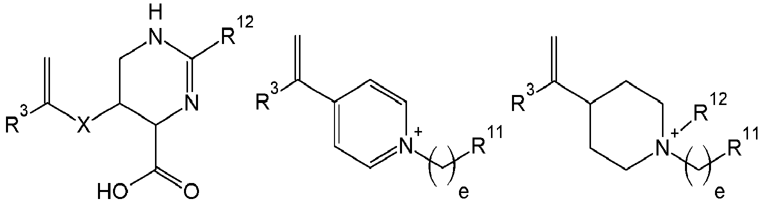 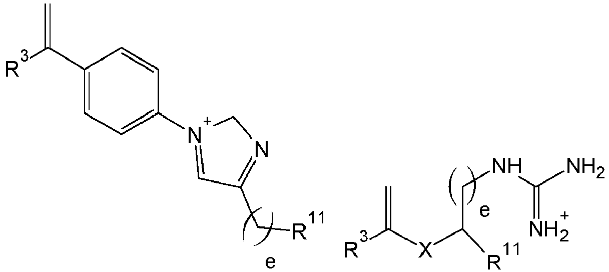 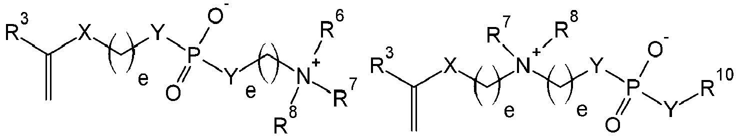 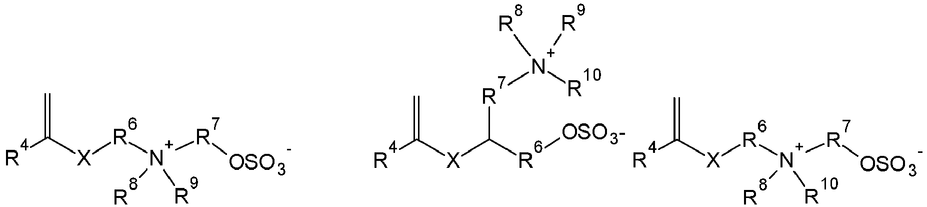 and 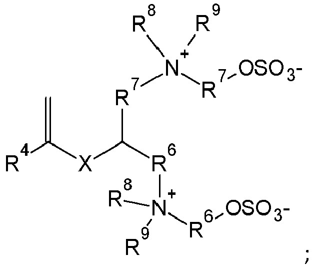
(b) optionally providing corsslinker monomers, optionally selected from the group consisting of 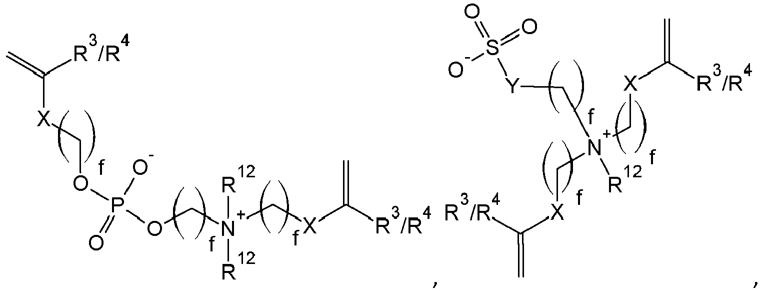 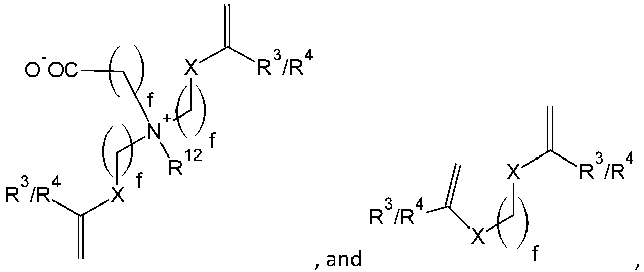 optionally in a molar fraction in the range of 0 to 99%, optionally 0 to 5%;
(c) reacting the monomers of step (a) and optionally (b) in a free radical polymerization or in a controlled radical polymerization, optionally using RAFT, ATRP or NMP in a suitable solvent;
(d) optionally functionalizing the polymer with R⁵ and/or R^{5'}, optionally in an esterification reaction, by click chemistry, amidation, thioesterification or by non-covalent modification; and
(e) isolating and optionally purifying the polymer.

The monomers for use in the present method can be any monomer as defined above or any monomer that corresponds to a residue defined for R¹ and R² in the context of the polymer for forming liquid droplets in an aqueous medium described herein. A monomer corresponding to a residue defined for R¹ and R² is a monomer wherein the entity 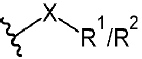 is replaced by 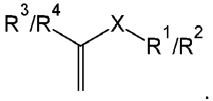 For example, the present invention also encompasses the method described herein for producing a polymer described herein in the context of the use of the polymer. For this method, the monomers used correspond to monomers which result in a residue defined for R¹ and R² in the context of the use of the polymer.

Reacting the monomers of step (a) and optionally (b) of the present method can also be achieved, e.g., using redox initiators or photoinitiators (for example, in combination with a UV source).

In step (c) of the present method, the suitable solvent may be an aqueous solvent, optionally a buffer selected from acetic buffer, aqueous buffer, mixtures of aqueous buffer with a water-miscible organic solvent, optionally ethanol, DMSO, DMF and THF, optionally a buffer with a salt concentration of about 500 mM to 3 M NaCl. Step (c) may optionally be performed in the absence of oxygen and presence of heat, optionally from 50 to 80 °C.

Functionalizing the polymer with R⁵ and/or R^{5'}, is to be understood as described in the context of the use described herein. R⁵ and/or R^{5'} may be a chemical entity resulting from a polymerization reaction as described herein, which entity is further chemically modified or functionalized (e.g. reacted with a TBM) to be a target binding moiety; or R⁵ and/or R^{5'} may be a target binding moiety in the absence of a chemical entity resulting from a polymerization. The term "functionalizing" means that a TBM is attached to the group by means of a chemical reaction to form, e.g. a covalent or ionic, bond with the chemical entity resulting from a polymerization. This attachment may result in the direct attachment of the TBM to the polymer under loss of the chemical entity resulting from a polymerization or it may result in the chemical modification of the chemical entity, e.g. partial loss of chemical entity. In step (d) of the present method, the following exemplary and non-limiting reactions for functionalizing the polymer can be made:
For an esterification between a carboxylic acid residue in the polymer with an amine-bearing targeting agent (e.g. antibody, antigen, protein A), the esterification can be performed in an aqueous buffer with EDC/NHS coupling.

For an azide-alkyne cycloaddition between an alkyne bearing polymer and an azide-bearing targeting agent such as, e.g. Biotin-azide (e.g. PEG4 carboxamide-6-azydoexanyl biotin), the reaction is not catalyzed by copper if DBCO is used as alkyne.

For a Michael addition between a thiol generated by the aminolysis of the carbonylthiol group of the RAFT agent and an alkene or maleimide-containing targeting agent (e.g. Biotin-maleimide), reference is made to H. Willcock et al. Polym. Chem., 2010, 1, 149-157.

The step of isolating and purifying in the present method can be done, e.g., via either dialysis or diafiltration against an aqueous buffer, via precipitation in a mixture of an aqueous buffer and organic solvent or via precipitation in an organic solvent.

In another aspect, the present invention is directed to a polymer for forming liquid droplets in an aqueous medium as described herein obtained or obtainable by the process described herein.

In an embodiment, the monomers described herein, including the monomer as defined above or any monomer that corresponds to a residue defined for R¹ and R² in the context of the polymer for forming liquid droplets in an aqueous medium as described herein, are polymerized to obtain the polymer for forming liquid droplets as described in any aspect of this disclosure.

In an embodiment, monomer(s) comprising residue R¹ and monomer(s) comprising residue R² are polymerized in a ratio of 0:1 to 100:1, optionally 0:1 to 10:1 or 0:1 to 1:1.

The following Figures and Examples serve to illustrate the invention and are not intended to limit the scope of the invention as described in the appended claims.

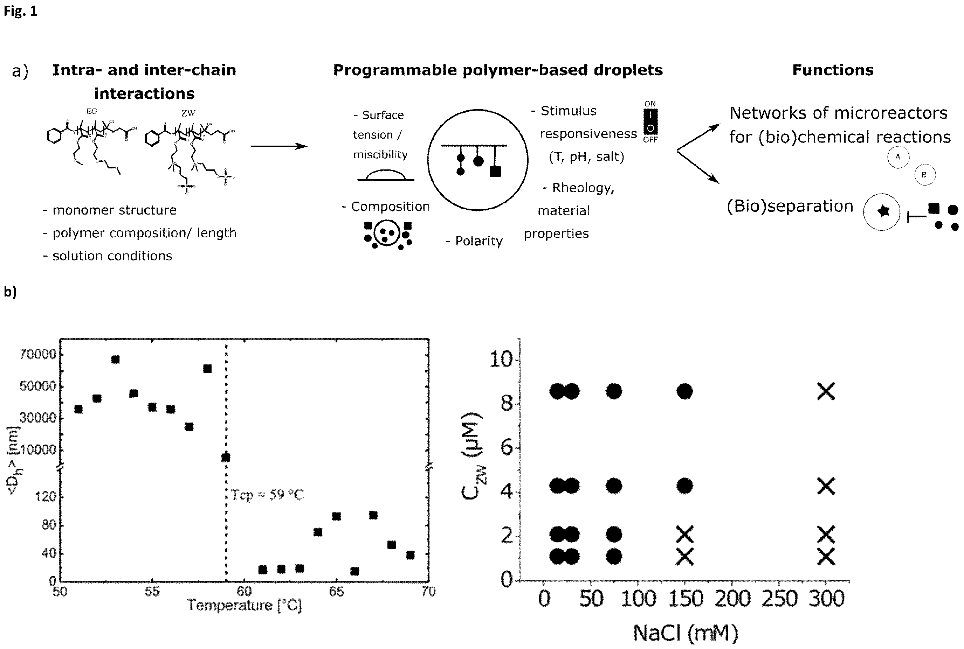

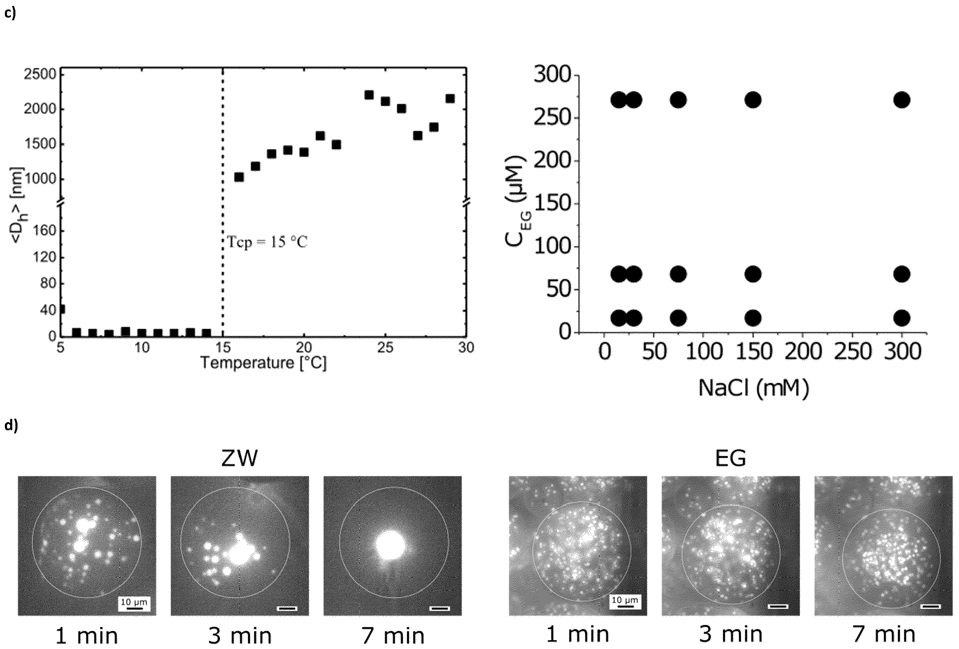

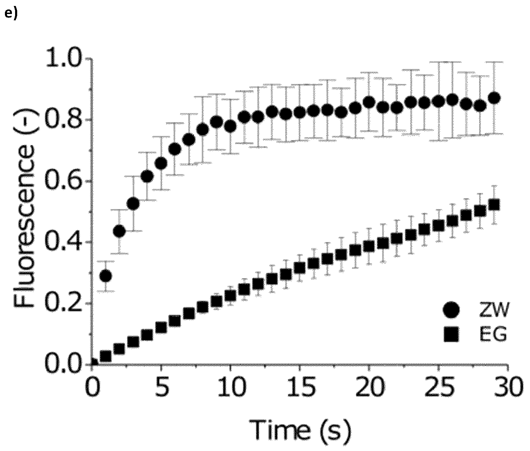

**Figure 1 a)** depicts a schematic illustration of the strategy of the invention. **b)** Formation of ZW coacervates as a function of temperature, as detected by measuring the average hydrodynamic radius by dynamic light scattering of 0.5 mg mL⁻¹ ZW aqueous solution at 30 mM NaCl and pH=7.5. Phase diagram of ZW at different polymer concentrations and ionic strengths. Red cross and black circles indicate absence and presence of phase separation. **c)** Hydrodynamic size of EG droplets as function of temperature. 0.5 mg mL⁻¹ EG in distilled water. Phase diagram of EG at different polymer concentrations and ionic strengths. Red cross and black circles indicate absence and presence of phase separation. **d)** Fusion of ZW droplets and (non fusion) of EG coacervates within microfluidic water-in-oil compartments; **f)** Fluorescence recovery after photobleaching (FRAP) of ZW and EG coacervates.

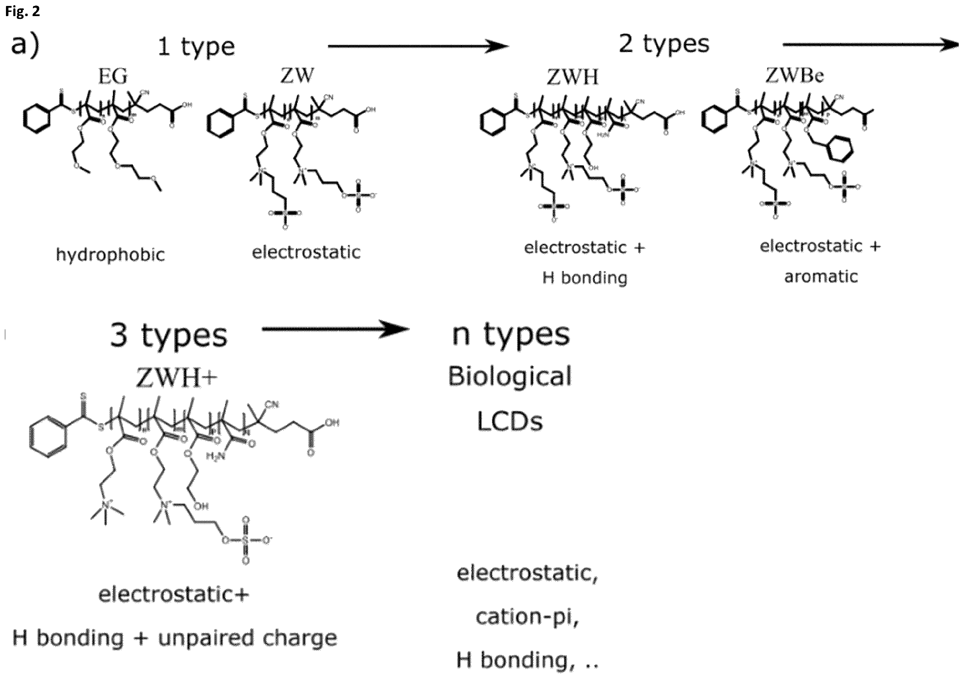

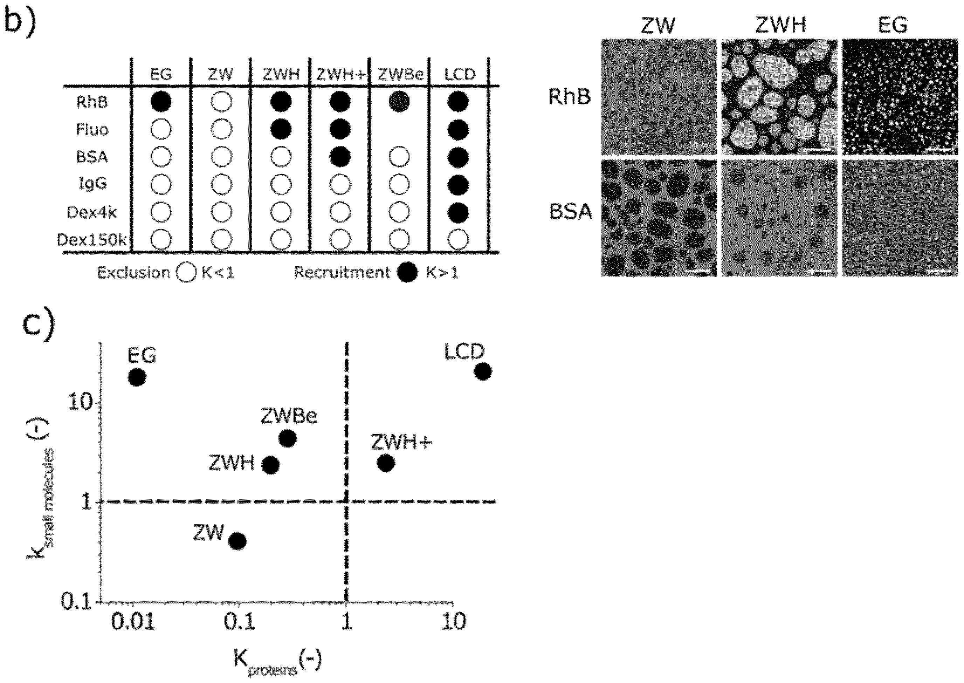

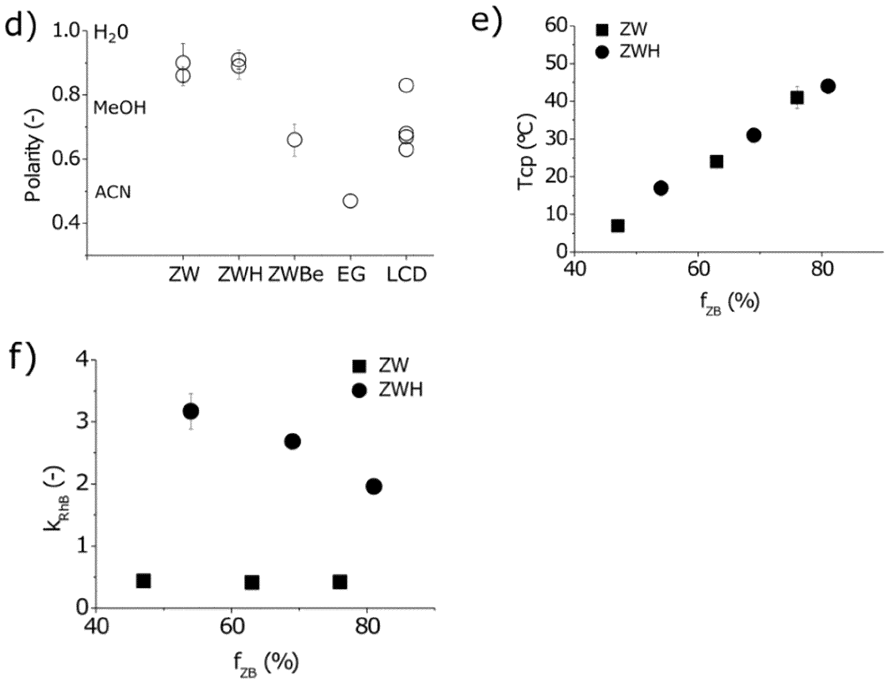

**Figure 2 a)** Structure of the synthesized polymers encoding a progressively increasing number of interactions; **b)** Partition coefficient of different molecules into coacervates of different copolymers and representative confocal images. **c)** Partition coefficient of Rhodamine and BSA in coacervates of different copolymers. **d)** Relative polarity with respect to water of the compartments made of different copolymers. **e)** Modulation of the cloud point temperature by modulating the composition of ZW and ZWH copolymers. **f)** Changes in the partition coefficient of Rhodamine by modulating the composition of ZW and ZWH copolymers.

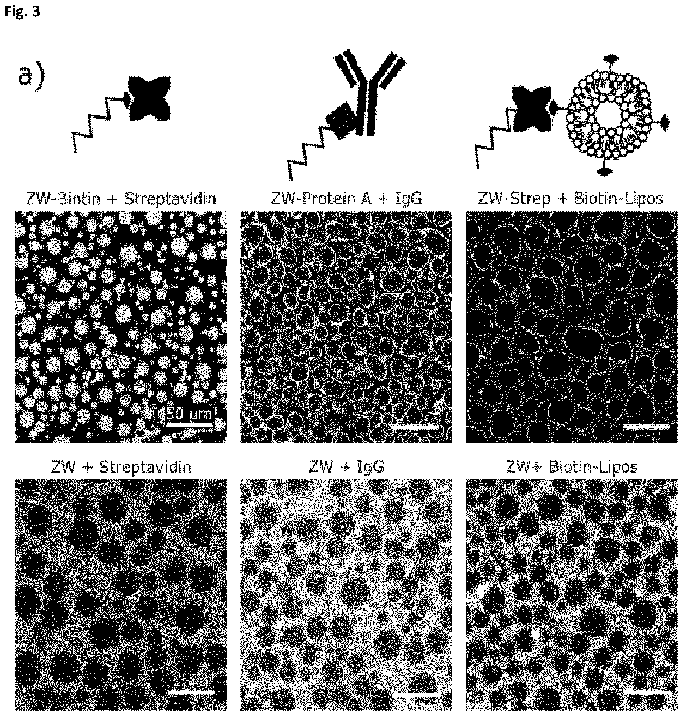

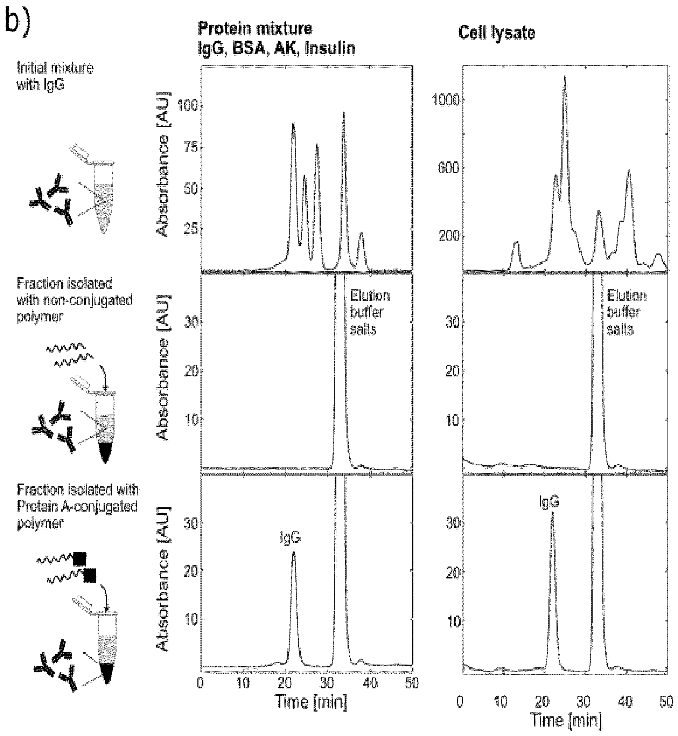

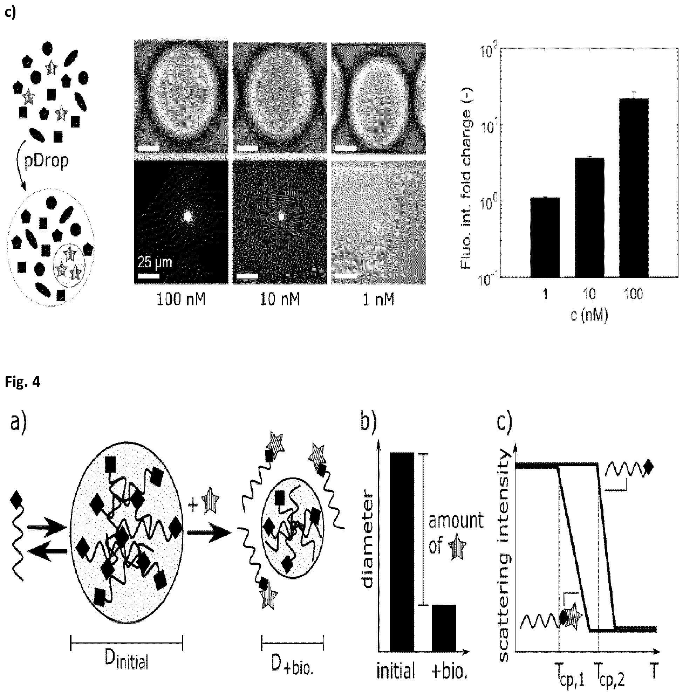

**Figure 3** **Programmable droplets for bioseparation. a)** Confocal images showing the recruitment of fluorescent streptavidin, IgG and liposomes into functionalized ZW-based droplets (upper panels). Lower panels show controls with compartments based on non-functionalized ZW polymers, showing the absence of uptake. **b)** Isolation of IgG molecules from a mixture of multiple proteins (bovine serum albumin (BSA), the enzyme adenylate kinase (AK) and insulin) and from a cell lysate. **c)** Detection assay implemented in a droplet-microfluidic platform to quantify tiny amounts of streptavidin in fetal bovine serum with the ZW-biotin functionalized polymers.
**Figure 4 a)** The liquid-liquid phase separation (LLPS) behavior of the ZW polymer changes upon binding to target analytes. For instance, the binding of the functionalized ZW to hydrophilic targets increases the solubility of the complexed ZW. If the uncomplexed polymer has a relatively low separating power, this binding leads to the migration of bound complexes outside the droplets, resulting in the shrinkage of the droplets and the reduction in the volume of the dispersed ZW phase. **b)** The shrinkage depends on the amount of hydrophilic target present. Using the change of solubility of ZW upon target binding, we can quantify the amount of target in solution, or the binding affinity of the conjugated ZW binding moiety and target. **c)** Other properties of the ZW change upon target binding, such as the Tcp which will decrease, and can also be monitored for the same applications suggested in b).
**Figure 5** Multiphase immiscible droplets. Depending on the interfacial energy, compartments of two different polymers can be fully miscible (**a**), fully immiscible (**b**) or form subcompartments, either with a core-shell morphology (**c**) or with partial wetting (**d**). The coexistence of multiple immiscible droplets with different physiochemical properties and with different responsiveness to external stimuli can be used to selectively recruit, purify, and detect different solutes at the same time from a single complex mixture.

### Example 1 - Materials

Methacrylamide (Mam, 98%, MW=85.10, Sigma Aldrich), 2-hydroxyethyl methacrylate (HEMA, 97%, MW = 130.14, Sigma Aldrich), 4,4'-azobis(4-cyanovaleric acid) (ACVA, ≥98%, MW = 280.28, Sigma Aldrich), 4-cyano-4-(phenylcarbonothioylthio)pentanoic acid (CPA, ≥97%, MW = 279.38, Sigma Aldrich), di(ethylene glycol) methyl ether methacrylate (95%, MW=188.22, Sigma Aldrich), ethylene glycol methyl ether methacrylate (95%, MW=144.17, Sigma Aldrich), N-(3-sulfopropyl)-N-methacroyloxyethyl-N,N-dimethylammonium betaine (SB, MW = 279.35, Merck), ethanol (≥99.8%, MW = 46.07, Sigma Aldrich), acetonitrile (ACN, 99.99%, MW = 41.05, Fisher Chemicals), 1,3-propanediol cyclic sulfate (TMS, 98%, MW = 138.14, Sigma Aldrich), sodium acetate (≥99%, MW=82.03, Sigma Aldrich), acetic acid (≥99%, MW=60.05, Sigma Aldrich) and sodium chloride (NaCl, ≥99.5%, MW = 58.44, Sigma Aldrich) were used as received. All solvents were of analytical grade purity and used without further treatment. Sulfabetaine methacrylate (ZB) was synthesized according to a previously published protocol (Sponchioni et al. Nanoscale, 2019,11, 16582-16591). Rhodamine B-based methacrylate (HEMA-RhB) was synthesized via DCC-mediated esterification of HEMA and Rhodamine B (RhB) according to a previously published protocol (ref). Briefly, 50 mg of RhB, 43 mg of DCC and 136 mg of HEMA were dissolved in 63 g of DCM and left to react overnight. The HEMA-RhB solution was then stored at 4°C.

### Example 2: Copolymers synthesis.

The copolymers were synthesized via RAFT copolymerization using ACVA as initiator and CPA as RAFT agent by modifying previously published protocols (Sponchioni et al. Nanoscale, 2019,11, 16582-16591; Sponchioni et al. J. Polym. Sci. Part A: Polym. Chem., 54: 2919-2931). In particular, all the sulfabetaine methacrylate (ZB)-based copolymers were synthesized in 20/80 vol/vol ethanol/ 3M NaCl acetic buffer (pH = 4.5) at 10 wt.% total monomer concentration. The CPA to ACVA molar ratio was set constant to 3 while the monomer to CPA molar ratios (i.e. the degree of polymerization of the single monomer *i* (*DP*ᵢ)) were varied according to **Table S1:**

**Table S1. Complete characterization of the synthesized polymers**

| Sample | *DP_{ZB}* [-] | *DP_{SB}* [-] | **DP*_{*HEMA*+*Mam*} [-] | *DP_{Be}* [-] | MN_{GPC} [g/mol] | *Ð*_{GPC} [-] | *f*_{ZB} [mol.%] | X [%] |
|---|---|---|---|---|---|---|---|---|
| ZW1 | 100 | 100 | - | - | 22048 | 1.1 | 47 | 98 |
| ZW2 | 130 | 70 | - | - | 21055 | 1.1 | 63 | 99 |
| ZW3 | 160 | 40 | - | - | 14544 | 1.2 | 76 | 99 |
| ZWH-1 | 100 | - | 100 | - | 17752 | 1.1 | 54 | 95 |
| ZWH-2 | 130 | - | 70 | - | 18942 | 1.2 | 69 | 97 |
| ZWH-3 | 160 | - | 40 | - | 19577 | 1.1 | 81 | 98 |
| ZWBe | 100 | 70 | - | 30 | 13847 | 1.1 | 47 | 99 |
| EG | - | - | - | - | 6335 | 1.2 | - | 93 |

| | | | | | | | | |
|---|---|---|---|---|---|---|---|---|
| *DP_{HEMA}=DP_{Mam} | | | | | | | | |

As an example, in the case of **ZW-2** (i.e the copolymer with *DP_{ZB}* = 130 and *DP_{SB}* = 70 according to **Table S1**), 3.36 g of SB, 6.6 g of ZB, 48 mg of CPA, and 14 mg of ACVA were dissolved in 40 g of 20/80 vol/vol ethanol/ 1M NaCl acetic buffer (pH = 4.5) and poured in a round bottom flask. The mixture was purged with nitrogen for 20 min and then heated to 65 °C for 24 h under constant stirring. The reaction mixture was dialyzed against 1 M NaCl for 3 days with a dialysis tubing (Spectra/Por^{®}, molecular weight cut-off (MWCO) = 3.5 kDa) by frequently changing the aqueous solution. The final polymer solutions were filtered with a 0.45 µm pore-size nylon membrane and stored at -20 °C. Copolymer concentrations were evaluated via gravimetry. ZB-based copolymers covalently labeled with Rhodamine B were synthesized by adding a previously synthesized Rhodamine B-based methacrylate (HEMA-RhB) in the initial reaction mixture. The same amount of monomer weight of HEMA-RhB solution was poured in a round bottom flask and the solvent was removed under nitrogen before the addition of the other reagents. The same synthesis and purification protocol were then applied. However, the dialysis was performed for at least 1 week to remove all the unbound HEMA-RhB. In a similar way, a primary amine functional group was introduced in the SB-based copolymers by adding AEMA in the initial reaction mixture by setting DP_{AEMA}=1 and by following the same synthesis and purification protocols.

In the case of **EG,** 52 mg of CPA, 10 mg of ACVA, 1.05 g of EG₂MA and 268 mg of EGMA were dissolved in 5.5 g of ethanol and poured in a round bottom flask. The mixture was purged with nitrogen for 20 minutes and left to react at 65°C for 2 days under constant stirring. The final copolymer was purified via precipitation in 45 mL of isopropyl ether. The recovered reddish waxy solid was dried under vacuum and stored at -20 °C. EG covalently labeled with Rhodamine B was synthesized by adding HEMA-RhB in the initial reaction mixture. 1.27 g of this solution was poured in a round bottom flask and the solvent was removed under nitrogen before the addition of the other reagents. The same EG synthesis and purification protocols were then applied.

### Example 3: Gel Permeation Chromatography.

The number-averaged molecular weight (Mn) and dispersity (*Ð*) of all the copolymers was evaluated via gel permeation chromatography. In the case of **EG,** a sample was dissolved at 4 mg mL⁻¹ in THF and filtered through a 0.45 µm pore-size PTFE membrane. The separation was performed on an Agilent set-up at a flow rate of 1 mL min⁻¹ at room temperature with a guard and two and two Agilent PLgel 20 µm MIXED-ALS columns. The values are reported in **Table S1** are relative to poly(methyl methacrylate) standards. In the case of all the other copolymers, samples were dissolved at 4 mg mL⁻¹ in 0.05 M Na₂SO₄/ acetonitrile (80/20 v/v) solution and filtered through a 0.45 µm pore-size nylon membrane. The separation was performed on an Agilent set-up at a flow rate of 0.5 mL min⁻¹ at room temperature with a guard and two Suprema columns (particle size 10 mm and pore sizes 100 and 1000 Å, Polymer Standards Service). The values are reported in **Table S1** and are relative to polyethylene glycol standards.

### Example 4: Nuclear Magnetic Resonance.

The conversion (*X*) and ZB molar fraction (*f_{ZB}*) of all the copolymers (**Table S1**) were evaluated via nuclear magnetic resonance (¹H-NMR) as reported in Sponchioni et al. Nanoscale, 2019,11, 16582-16591. An aliquot of all the reaction mixtures were withdrawn before and after the reaction completion. The samples were dried under nitrogen, dissolved in either 3 M NaCl D₂O for all the SB-based copolymers or CDCl₂ for EG copolymers and analyzed on a Bruker NMR.

### Example 5: Cloud point.

Cloud points of the polymer droplets (T_{cp}, **Table S2**) were evaluated via dynamic light scattering (DLS) at 0.25 mg mL⁻¹ polymer concentration and at 150 mM NaCl. 200 µL of the sample were let to equilibrate at 65°C for 20 minutes before analysis. The scattered light and droplet size was then measured from 65°C to 5°C with a temperature step of 1°C and an equilibration time of 10 min. The cloud point was considered as the inflection point of the curve.

**Table S2. Characterization of the polymer droplets**

| Sample | *T_{cp}* [°C] | *C_{m,salt}* [mM] | *W*_{*con*t} [wt.%] | *ρ* [g ml⁻¹] | *γ* [mN m^{- 1}] | *k_{RhB}* [-] | *k_{BSA}* [-] | *Polarity* [-] |
|---|---|---|---|---|---|---|---|---|
| ZW1 | 7 ± 1 | 95 ± 5 | 48 ± 1 | 1.27 ± 0.07 | 77 ± 1 | 0.441 ± 0.001 | 0.11 ± 0.01 | 0.90 ± 0.06 |
| ZW2 | 24 ± 1 | 185 ± 5 | 53 ± 2 | 1.23 ± 0.05 | 81 ± 4 | 0.414 ± 0.004 | 0.13 ± 0.01 | 0.90 ± 0.06 |
| ZW3 | 41 ± 3 | 305 ± 5 | 45 ± 6 | 1.33 ± 0.10 | 78 ± 6 | 0.423 ± 0.004 | 0.07 ± 0.01 | 0.86 ± 0.03 |
| ZWH1 | 17 ± 2 | 125 ± 5 | 53 ± 4 | 1.16 ± 0.06 | 58 ± 2 | 3.172 ± 0.289 | 0.31 ± 0.01 | 0.89 ± 0.04 |
| ZWH2 | 31 ± 1 | 270 ± 5 | 53 ± 6 | 1.30 ± 0.01 | 74 ± 4 | 2.682 ± 0.062 | 0.18 ± 0.07 | 0.89 ± 0.01 |
| ZWH3 | 44 ± 2 | 395 ± 5 | 46 ± 3 | 1.31 ± 0.05 | 78 ± 1 | 1.956 ± 0.096 | 0.15 ± 0.02 | 0.91 ± 0.03 |
| ZWBe | 45 ± 1 | 325 ± 5 | 50 ± 3 | 1.23 ± 0.05 | 68 ± 1 | 4.563 ± 0.570 | 0.28 ± 0.01 | 0.66 ± 0.05 |
| EG | 19 ± 1 | - | 16 ± 2 | 1.21 ± 0.06 | 18 ± 9 | 18.99 ± 1.54 | 0.01± 0.03 | 0.47 ± 0.02 |

### Example 6: Critical salt concentration.

The critical salt concentrations (*C_{m,salt},* **Table S2**) at which the polymer droplets disappear were evaluated via wide-field microscopy using a dichotomous search method. 0.25 mg mL⁻¹ of the polymer aqueous solutions at different NaCl concentrations were incubated at room temperature in a 384-well plate (MatriPLate, Glass Bottom, Brooks) and imaged after 24 hours.

### Example 7: Physical characterization of the polymer dispersed phase.

Pure polymer-rich phases were obtained via dialysis of polymer solutions against distilled water at room temperature for 2 days with a membrane tubing (MWCO = 3.5 kDa, SpectrPor^{®}). The dispersed phases were collected at the bottom of the membrane tubing and stored wet at 4°C. The water content (*W_{cont}* in **Table S2**) and density (*ρ* in **Table S2**) of the polymer dispersed phases were evaluated via gravimetry of known volume samples. The interfacial tension (*γ* in **Table S2**) was evaluated with a contact angle system OCA 15 plus. The polymer phase was loaded in a 1 mL syringe loaded with a 25G gauge needle with a flat tip. The interfacial tension was evaluated using the SCA20 software (dataphysics).

### Example 8: Droplet Polarity.

The polarity of the droplets (*Polarity* in **Table S2**) was measured using PRODAN as solvatochromic dye according to a previously published protocol (A. M. Küffner et al. ChemSystemsChem 2020, 2, e2000001). Briefly, the emission spectrum of PRODAN was acquired in different droplets between 400 and 600 nm with a 5 nm wide spectral window using a fluorescence confocal microscope. The wavelength that corresponds to the maximum fluorescent emission was used to evaluate the relative polarity of the droplets by applying a linear fit of reference solvents with different polarities (see C. C. Vequi-Suplicyet al. J. Fluoresc. 2015, 25, 621- 629; O. A. Kucherak et al. J. Phys. Chem. Lett. 2010, 1, 616- 620).

### Example 9: Droplet Liquidity.

The liquidity of the droplet was evaluated via fluorescence recovery after photo-bleaching (FRAP) according to a previously published protocol (Nicole O. Taylor et al., Biophysical Journal, 117, 7, 2019, 1285-1300). 5 mg mL⁻¹ of RhB covalently labeled-polymer in 90 mM NaCl aqueous solutions were incubated at room temperature for 24 hours and then photo-bleached at 561 nm. The photo-bleached spot radius was set equal to 1/10 of the droplet radius. Result are an average of at least three different droplets. Droplet fusion events were also monitored on chip.

### Example 10: Protein synthesis and characterization.

DDX4 (LCD) wasp roduced in E.coli BL21-GOLD (DE3) cells. Protein production was induced at optical density (OD) around 0.7 with 0.5 mM isopropyl d-thiogalactopyranoside (99%, PanReac AppliChem). After 16 h at 37 °C, the recombinant proteins were purified using His-tag immobilized metal ion affinity chromatography (Chelating Sepharose, GE Healtchare). The proteins were further purified via size exclusion chromatography using a Superdex 75 26/600 on an AKTA Prime system (GE Healthcare) in 50 mM Tris at pH =7.5 and 500 mM NaCl. The quality of the purified proteins was confirmed by SDS-PAGE electrophoresis. The proteins were concentrated to 600-700 µM and aliquots were frozen and stored at -20 °C.

**Table S3. Partitioning coefficient of different molecules and macromolecules in droplets constituted by different polymers**

| | EG | ZW2 | ZWH1 | ZWH+ | ZWBe | LCD |
|---|---|---|---|---|---|---|
| Rhodamine B | 18.99 ± 1.54 | 0.414 ± 0.004 | 3.172 ± 0.289 | 1.72 ± 0.04 | 4.563 ± 0.570 | 22.22 ± 1.6 |
| Fluorescein | 0.31 ± 0.1 | 0.305 ± 0.05 | 1.97 ± 0.04 | 2.44 ± 0.24 | | 3.66 ± 0.02 |
| BSA | 0.01 ± 0.03 | 0.13 ± 0.1 | 0.31 ± 0.01 | 3.05 ± 1.4 | 0.28 ± 0.01 | 27 ± 5 |
| IgG | 0.39 ± 0.15 | 0.19 ± 0.01 | 0.35 ± 0.17 | 0.4 ± 0.02 | 0.14 ± 0.02 | 1.54 ± 0.12 |
| Dextran 4kDa | 0 ± 0.01 | 0.08 ± 0.02 | 0.04 ± 0.05 | 0.2 ± 0.03 | 0.01 ± 0.01 | 1.62 ± 0.01 |
| Dextran 150kDa | 0.48 ± 0.01 | 0.33 ± 0.08 | 0.34 ± 0.13 | 0.58 ± 0.01 | 0.40 ± 0.14 | 0.67 ± 0.01 |

### Example 11: Evaluation of solute partition coefficients.

The partition coefficient of the solutes between different phases was evaluated via confocal fluorescence microscopy as K = (I _{droplet} - I_{background}) / (I_{bulk} - I_{background}) where I _{droplet} and I _{bulk} are the average fluorescence intensity inside and outside the droplets, respectively, and I_{background} is the average fluorescence intensity of the samples at the same conditions, but without the presence of any fluorescent solute. The values reported are an average of at least three different micrographs.

### Example 12: Polymer conjugation.

23.4 mg of 50SB-150ZB, 7.2 mg NHS and 4.8 mg EDC were dissolved in 500 µL MES buffer (0.1 M MES, 1 M NaCl, pH 6.0) and reacted at room temperature for 15 min. The pH was raised to 7.2 with 1 M Na2CO3 and a solution of either 2.4 mg biotin-PEG7-NH2 or 4 mg Protein A in 50 µL coupling buffer (0.1 M Na-phosphate, 1 M NaCl, pH 7.2) was quickly added and gently shaken at room temperature for 1 hour. Then, ethanolamine was added to the solution to achieve a final concentration of 1 M and shaken at room temperature for 30 min. The solutions were diluted 30 times in Millipore water and kept at 4°C for at least 2 hours to promote phase separation. The solution was centrifuged at 6000 x rcf for 10 minutes. The supernatant was removed and the solution was resuspended in 500 µL 5x PBS or 5x Protein A equilibration buffer (0.1 M Na-phosphate, 0.75 M NaCl, pH 7.5), respectively. The solutions were then diluted, incubated and centrifuged as before. The separated polymer conjugates were then resuspended to achieve theoretical final polymer concentrations of 200 mg/mL. Similarly, 23.4 mg 10SB-190ZB were activated with NHS esters. Then, 15 µL of the polymer solution at pH 7.2 were added to 100 µg streptavidin in 10 mM phosphate (10 µL) and 2.5 µL high salt buffer (5 M NaCl, 2.7 mM KCI, 10 mM Na2HPO4, 1.8 mM KH2PO4, pH 8.8) and shaken at room temperature for 1 hour. The solution was then mixed, shaken with ethanolamine and washed as previously described. 5x PBS with 1 M NaCl was used as resuspension buffer.

### Example 13: Microscope analysis of bioseparation.

Biotin-streptavidin: 1 µg streptavidin-ATTO425 was premixed with 37 µg BSA, 74 µL PBS and 20 µL Millipore water. Then, approximately 25 µg (5µL) of either 50SB-150ZB-biotin or 50SB-150ZB (control) in 5x PBS was added to the well and let phase separate at room temperature. Protein A-IgG: 6 µg IgG-ATTO647N was premixed with 80.5 µL Protein A equilibration buffer and 14 µL Millipore water. Then, approximately 87 µg (3.5 µL) of either 50SB-150ZB-Protein A or 50SB-150ZB (control) in 5x Protein A equilibration buffer was added to the well and let phase separate at room temperature. Streptavidin-biotinylated liposomes: biotinylated-rhodamine B liposomes were premixed with 47 µL PBS and 45 µL Millipore water. Then, approximately 25 µg (3.5 µL) of either 10SB-190ZB-streptavidin or 10SB-190ZB (control) in 5x PBS with 1 M NaCl was added to the well and let phase separate at room temperature.

### Example 14: Purity of bioseparated species.

A protein solution was obtained spiking 1200 µL Millipore water and 500 µL Protein A equilibration buffer (20 mM Na-phosphate, 0.15 M NaCl, pH 7.5) with 100 µg bovine serum albumin (BSA), 100 µg adenylate kinase (AK), 100 µg insulin, 80 µg Immunoglobulin G (IgG) and, when specified, with 500 µL cell lysate. 80 µL of a 134 mg/mL 10SB-190ZB or Protein A-conjugated 10SB-190ZB solution were added to 735 µL of the protein solution, quickly vortexed and incubated at room temperature for 15 minutes and at 4°C for 15 minutes. The samples were centrifuged at 3000 rcf and 4°C for 1 minutes and the supernatants were removed. The polymer phases were first dissolved in 100 µL 7 x protein A equilibration buffer (0.14 M Na-phosphate, 1.05 M NaCl, pH 7.5), then diluted in 600 µL Millipore water and incubated at 4°C for 15 minutes. The samples were centrifuged as before and the supernatants removed. The polymer phase was dissolved in 150 µL high salt elution buffer (0.1 M citric acid, 0.5 M NaCl, pH 3.0), diluted with 300 µL low salt elution buffer (0.1 M citric acid, pH 3.0) and incubated at 4°C for 15 minutes and centrifuged as before. The elution supernatants were then collected and analyzed by size exclusion chromatography after increasing their pH to 6.3 with 1 M Tris buffer at pH 9.0.

### Example 15: Microfluidics.

To monitor the size distributions over time as well as the UCST and LCST behavior of the ZW2 and the EG polymer, the droplets were encapsulated into water-in-oil emulsions using a microfluidic device with a flow focusing geometry. The microfluidic devices were fabricated as previously described (ML, Angew. Chem. 58 (41), 2019, 14498-14494). In brief, a master wafer was fabricated by spin-coating SU-8 photoresist polymer (MicroChem) on a silicon wafer. Afterwards, a mask was applied on top of the polymer layer, containing the desired pattern of the channels. After UV polymerization, hardening and developing, the desired channel structure was left as relief on the master wafer. The chips were realized by standard soft lithography: polydimethylsiloxane (PDMS) was mixed with curing agent (Sylgard 184, Silicone Elastomer Base / Curing Agent, Dow Corning) at a ratio of 10:1, poured on the master wafer, degassed and cured for 30 min at 70 °C. The same procedure was carried out with a blank master wafer without channel structures to fabricate a thin PDMS bottom layer (about 2 mm). To obtain functional channels, the two layers were laid on top of each other and inter-connected by further crosslinking over night at around 110 °C. In case of the ZW2, the phase transition was induced on chip. The chip contains three inlets and one outlet as well as two junctions where fluids can be mixed in a highly controllable manner. At the first junction, the homogenous polymer solution (10 mg/ml stock in 500 mM NaCl) was mixed with H2O with a flow rate ratio of 2:1 (0.4 µl/min and 0.2 µl/min, respectively are absolute flow rates) using syringe pumps (Cetoni, neMESYS, Cetoni GmbH). This induced a rapid drop in the salt concentration, resulting in the formation of liquid-like, phase separated polymer droplets at 3.3 mg/ml polymer in 166.67 mM NaCl. These droplets were then encapsulated into water-in-oil compartments at a second junction, by mixing them with HFE-7500 oil (Acota Limited) supplied with 0.2 % surfactant (Pico-Surf 1, Sphere Fluidics, Cambridge, UK). After formation, the compartments containing the phase-separated droplets were transferred into capillaries (0.1 mm inner diameter; CM Scientific) and observed off chip. Droplet coalescence was observed over a time scale of 8 min until only a single droplet was left in each compartment. To heat the encapsulated phase-separated droplets, the capillaries were transferred to a glass slide, covered with a thermo-conductive coating and connected to a power supply (RND-320 KD3005D, Distrelec). Applying voltage ranging from 0 to 3 V increased the temperature from around 25°C to around 40°C, which was measured by a thermocouple (NI 9211, National Instruments) connected to the NI MAX LABView software. While heating / cooling the sample, the disappearance and re-appearance of the droplets was monitored over time. The encapsulation and heating of the RhB-labeled polymers were observed on a Nikon Eclipse Ti-E epifluorescence microscope. The RhB-labeled polymers were excited (λexc = 546 nm) with a LEDHub light source (Omicron LedHUB Light engine) and detected (λexc = 568 nm) using an Andor Zyla sCMOS camera. For the EG polymer, the phase separation was induced off chip by equilibrating a solution of 1.25 mg/ml polymer at room temperature. The resulting droplets were encapsulated into water-in-oil compartments and subsequently transferred into capillaries. To cool the samples, an in-house built cooling stage was used to decrease the temperature of the encapsulated droplets to 14.5 °C, below the Tcp of the polymer. Images were taken as described above. Image analysis was carried out by using a Matlab code to extract droplets radii over time.

### Example 16: In-situ analysis of a biomarker via microfluidics

In bulk, the ZW-droplet assay has a theoretical upconcentration limit of 10x, considering an initial sample of 200 µL and a final ZW condensed phase enriched in the target analyte of 20 µL. In order to achieve a much higher concentration factor, we implemented the assay in a microfluidic format. In this case, the shrinkage of the compartments is replaced by the formation of a polymer-rich droplet within the compartments. Assuming a microcompartment size of 100 µm diameter and a polymer-rich droplet upconcentrating the analyte of less than 5 µm, we could achieve a concentration factor of approximately 10'000x. As model analytes ZW conjugated to Biotin were used to recruit fluorescently labeled streptavidin (ATTO 425-Streptavidin, 09260, Sigma Aldrich). The microfluidic device used for droplet generation consisted of two polydimethylsiloxane (PDMS, Silicone elastomer 184, Sylgard 184 kit, Dow Corning, USA) layers bonded to each other. The first layer was thick (approximately 10 mm) and contained the droplet generation module with a nozzle measuring 50 µm width and a channel with a 1600 µm wide and 9500 µm long geometry. The mold corresponding to this layer was prepared by spin-coating SU-8 photoresist on silica wafers (MicroChemicals, Germany). Selected regions of the coated wafer were exposed to UV light for curing. The second layer consisted of a flat and thin (<1mm) PDMS layer. Standard soft-lithography was used to replicate the channels and to cast the flat PDMS layer on a flat wafer. PDMS mixed with the corresponding cross-linker at a 15:1 weight ratio was cast on the molds and degassed under vacuum at 10 mbar for 30 min. Partial curing of these PDMS layers was achieved by incubation for 25 min at 70°C. After peeling off the PDMS layers from the wafers, the channel layer was aligned and contact bonded to the flat layer. Full bonding of the two layers and complete curing of the PDMS were achieved by incubating the device at 60°C overnight. Water-in-oil droplets were generated in this device by emulsifying the analyte and polymer droplet containing solutions in Fluoridrop 7500 oil (Dolomite Microfluidics, United Kingdom) with 0.1% Picosurf 1 (Dolomite Microfluidics) as surfactant. The flow in the channels was imposed with syringe pumps (Cetoni neMESYS, Cetoni GmbH, Germany). The droplets generated on the device were collected in square quartz capillaries (0.1 mm inner diameter, CM Scientific, UK) and incubated for 30 min to allow analyte recruitment and the merging of all ZW droplets into a single LLPS phase. The droplets were then imaged on Ti2-U epi-fluorescence inverted microscope (Nikon, Switzerland) equipped with an automatic stage, LED light sources operating at 455 nm and 617 nm (Omicron Laserage Laserprodukte GmbH, Germany), and a camera (Zyla sCMOS 4.2P-CL10, Andor, United Kingdom). The different analytes were detected with filter cubes sets (AHF Analysentechnik AG, Germany) at the following excitation/emission wavelengths: CFT ET filter set (436 nm - 480 nm) for the labeled streptavidin, and a Cy5 ET Filter set filter set (640 nm/690 nm) for the ATTO647N tagged detection antibodies. Droplets were imaged with a 20x magnification objective (CFI Plan Apo Lambda 20X, Nikon, Switzerland). Bulk samples were prepared in 384-well plates (MatriPlate 384Well Plate, Glass Bottom, Brooks). The recruitment of labeled streptavidin was tested by ZW-Biotin, and Aβ42 by ZW-HET, where HET corresponds to an Aβ42 antibody binding fragment.

## Claims

1. A use of liquid droplets in an aqueous medium for the controlled uptake of compounds from outside the droplets into the droplets for separation, storage and/or reaction of compounds inside the droplets, wherein the droplets comprise a polymer according to Formula (I) 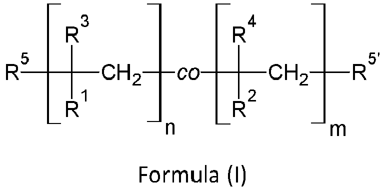 wherein
**n** is an integer selected from 0 to 2500, optionally from 25 to 250;
**m** is an integer selected from 2 to 2500, optionally from 25 to 250;
**R³** and **R⁴** are each independently selected from the group consisting of hydrogen and methyl;
**R⁵** and **R^{5'}** are independently selected from
- hydrogen, linear or branched (C₁₋₁₀)alkyl and phenyl;
- a group resulting from a polymerization initiator, optionally a group resulting from a RAFT polymerization initiator, optionally 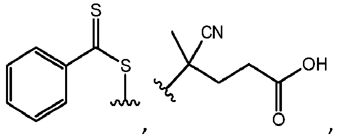 or
- a target binding moiety (TBM), optionally a TBM attached to or reacted with a group resulting from a polymerization initiator, optionally from a RAFT polymerization initiator, optionally 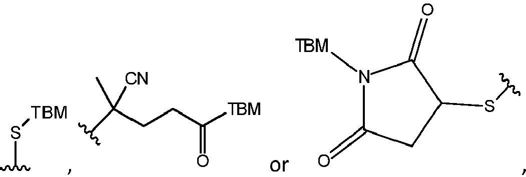 wherein the TBM is optionally selected from the group consisting of azide, alkyne, carboxylate, amine, thiol, hydroxyl, aldehyde, cyanate, sulfonyl, tosyl, tresyl, epoxide, carbonate, halide, anhydride, carbamate, imidazole, azlactone, triazine, maleimide, aziridine, peroxide, acyl, anthraquinone, diazo, diazirine, psoralen, NHS ester, imido ester, (C₂₋₁₀)alkenyl or alkyl groups, DBCO, cytosine, guanidine, streptavidin, avidin, biotin, an aptamer, a peptide, a protein, an oligonucleotide, optionally a hydrophilic uncharged oligopolymer, a positively or negatively charged oligopolymer, a nucleic acid, a carbohydrate, a dye, a cell, an antibody, an organic and inorganic nanoparticle, hydrophilic uncharged oligopolymers, positively and negatively charged oligopolymers;
**R¹** is selected from the group consisting of
(i) -X-hydrogen, -X-methyl, -X-ethyl and -X-propyl; 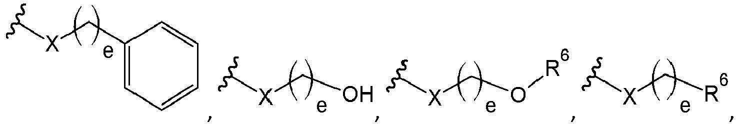
(ii) 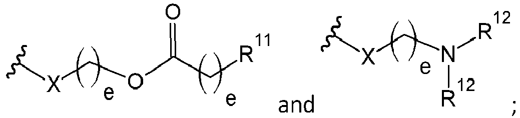
(iii) 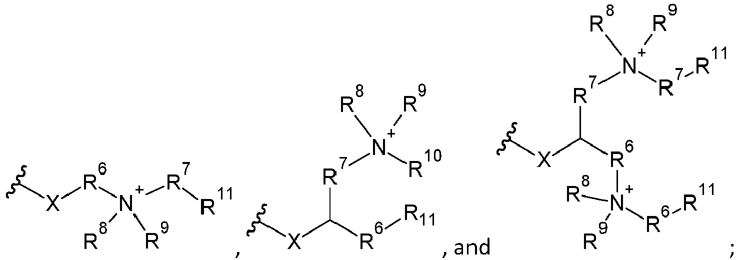
(iv) 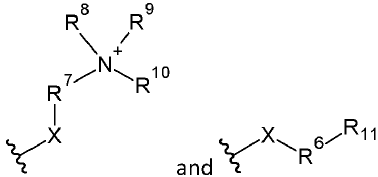 in a ratio of about 1:1 within the polymer;
(v) 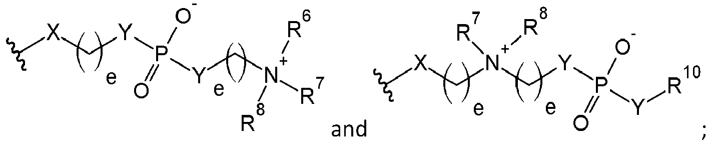 and
(vi) 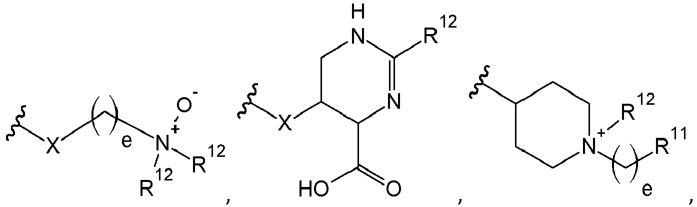 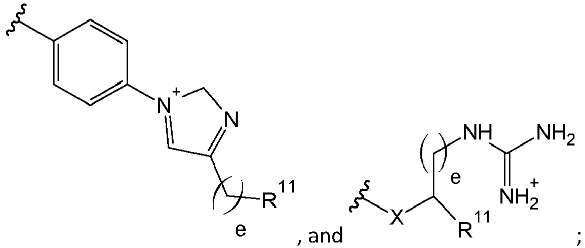
**R²** is selected from the group consisting of 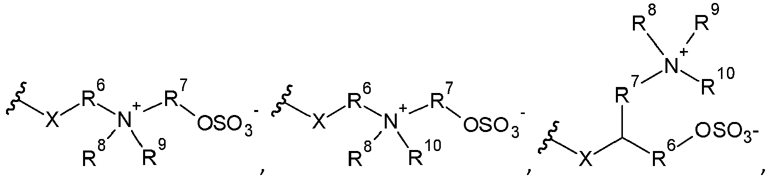 and 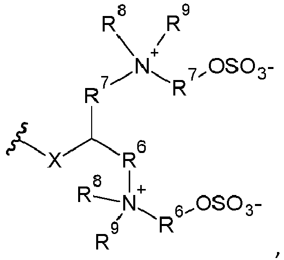 wherein
**X** is selected from the group consisting of -C(=O)-O-, -C(=O)-NH-, aliphatic and aromatic carbo- or heterocycle;
**Y** is oxygen or absent;
**e** is an integer selected from 1 to 5, optionally from 2 to 3;
**R⁶** and **R⁷** are each independently selected from the group consisting of
linear or branched, substituted or non-substituted (C₁₋₁₀)alkyl, optionally (C₁₋₅)alkyl, optionally methyl, ethyl and propyl,
linear or branched, substituted or non-substituted (C₁₋₁₀)alkyl, optionally (C₂₋₆)alkyl chains, comprising one or more heteroatoms selected from the group consisting of O, N, S and P, optionally (C₂₋₅)alkyl ethers,
(C₂₋₁₀)alkenyl,
phenyl, and an aromatic heterocycle, optionally pyridine, pyrrole, furan and imidazole;
**R⁸, R⁹** and **R¹⁰** are each independently selected from the group consisting of hydrogen, linear or branched, substituted or non-substituted (C₁₋₁₀)alkyl, optionally (C₁₋₅)alkyl, optionally methyl, ethyl and propyl, (C₂₋₁₀)alkenyl, phenyl, an aromatic heterocycle, optionally pyridine, pyrrole, furan and imidazole, 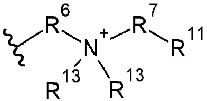 when forming R¹, 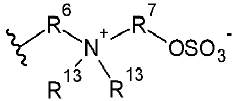 when forming R²;
**R⁸** and **R⁹** together or two **R¹³** together optionally form an aliphatic or aromatic heterocycle, optionally a piperidine ring, a piperazine ring or a morpholine ring;
**R⁸, R⁹** and **R⁶** or **R⁸, R⁹** and **R⁷** optionally together form a five or six-membered ring, optionally a pyridine ring, a piperidine ring, pyrrole ring, a pyrimidine ring, a pyrazole ring, an imidazole ring, a pyrazine ring, an isoxazole ring or an oxazole ring; wherein one of **R⁸** or **R⁹** is absent if **R⁸** and R⁶, **R⁹** and **R⁶** or **R⁸** and **R⁷,** or **R⁹** and **R⁷** together form an aromatic ring;
**R¹¹** is selected from the group consisting of -SO₃⁻, -C(=O)O⁻, and 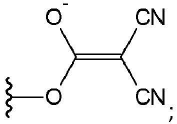
**R¹²** is selected from the group consisting of methyl, ethyl and propyl;
**R¹³** is independently selected from the group consisting of linear or branched, substituted or non-substituted (C₁₋₁₀)alkyl, optionally (C₁₋₅)alkyl, optionally methyl, ethyl and propyl, (C₂₋₁₀)alkenyl, phenyl, an aromatic heterocycle, optionally pyridine, pyrrole, furan and imidazole,
wherein the polymer optionally is crosslinked by a crosslinker, optionally a crosslinker as R¹.

2. The use according to claim 1, wherein
**n** is an integer selected from 10 to 250;
**m** is an integer selected from 10 to 250;
**e** is an integer selected from 2 to 3;
**X** is selected from the group consisting of -C(=O)-O-, -C(=O)-NH-, pyridine, piperidine and phenyl;
**R⁶** and **R⁷** are each independently selected from the group consisting of (C₁₋₅)alkyl, optionally methyl, ethyl and propyl;
**R⁸, R⁹** and **R¹⁰** are each independently selected from the group consisting of methyl, 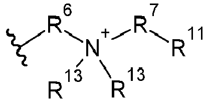 when forming R¹, and 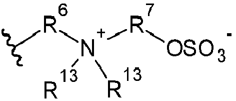 when forming R²; **R¹¹** is -SO₃⁻ or -C(=O)O⁻;
**R¹²** is methyl; and
**R¹³** is selected from the group consisting of methyl, ethyl and propyl.

3. The use according to claim 1 or 2, wherein
**R¹** is selected from the group consisting of
(i) 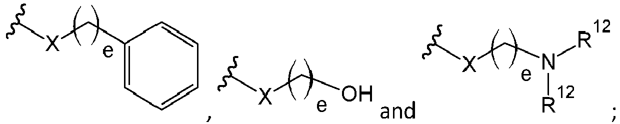
(ii) 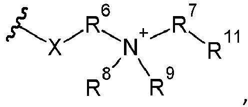 optionally 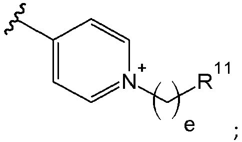
(iii) 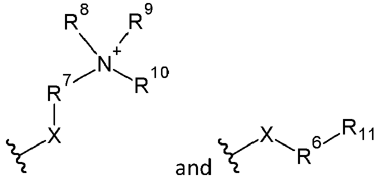 in a ratio of about 1:1 within the polymer;
(iv) 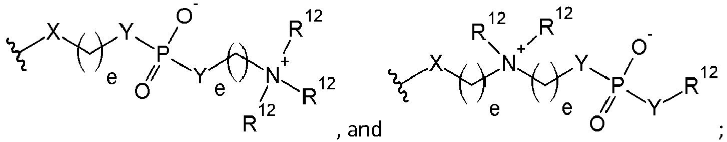 and
(v) 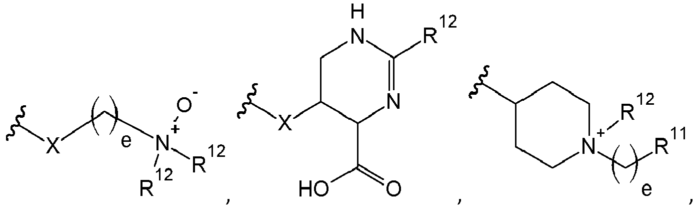 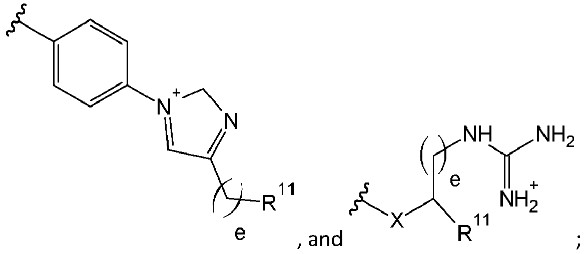 and **R²** is

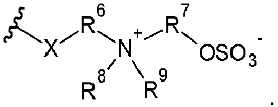

4. The use according to any of claims 1 to 3, wherein
**n** is an integer selected from 10 to 250;
**m** is an integer selected from 10 to 250;
**R⁵** and **R^{5'}** are hydrogen or independently selected from the group consisting of azide, alkyne, carboxylic acid, amine, (C₂₋₁₀)alkenyl, streptavidin, avidin, biotin, an aptamer, a peptide, a protein, an oligonucleotide, a cell, an antibody, a hydrophilic uncharged oligopolymer, a positively or negatively charged oligopolymer and an organic or inorganic nanoparticle;
**R¹** is selected from the group consisting of
(i) 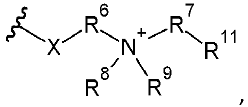
(ii) 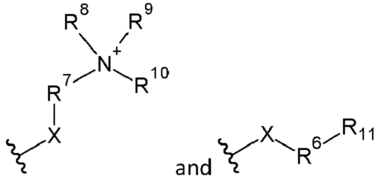 in a ratio of about 1:1 within the polymer;
(iii) 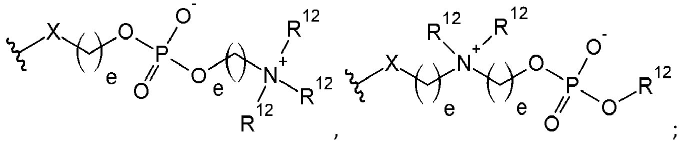 and
(iv) 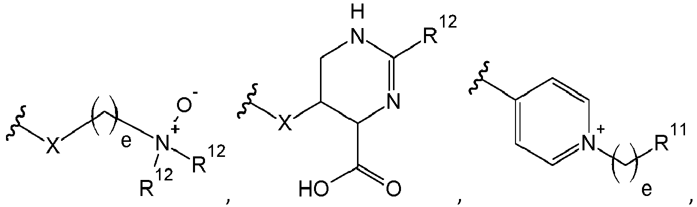 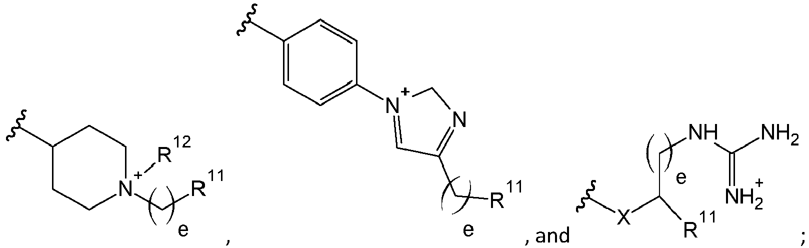 **R²** is 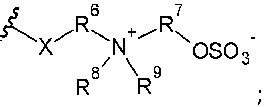 wherein
**R⁶** and **R⁷** are each independently selected from the group consisting of methyl, ethyl and propyl;
**R⁸, R⁹, R¹⁰, R¹²** and **R¹³** are each methyl; and
**R¹¹** is -SO₃⁻ or -C(=O)O⁻.

5. The use according to any of claims 1 to 4, wherein
**R¹** is selected from the group consisting of 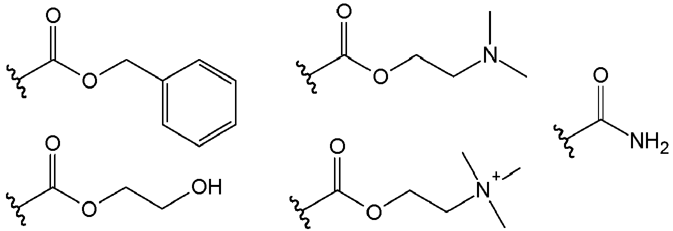 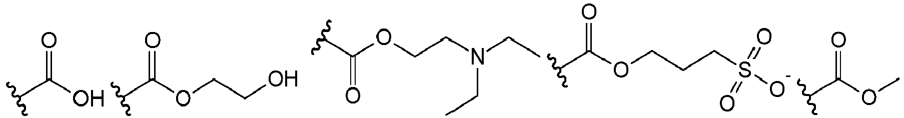 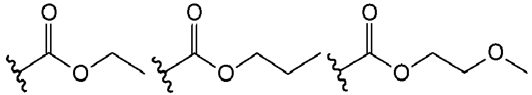 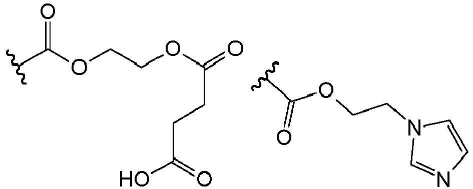 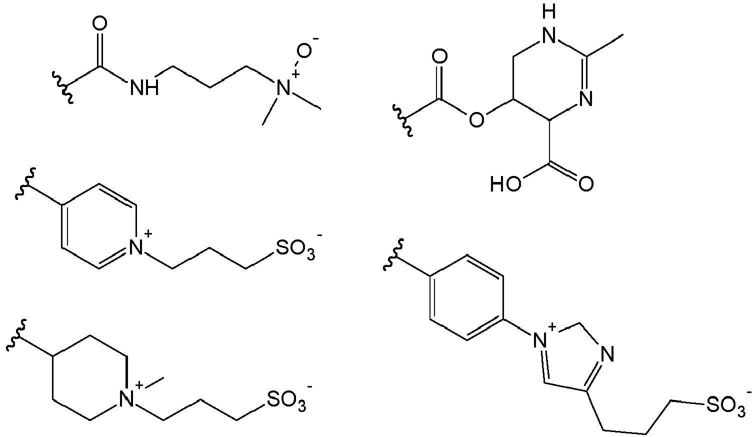 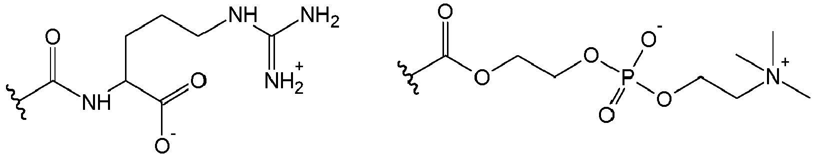 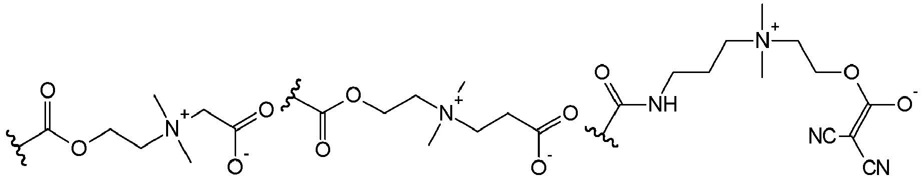 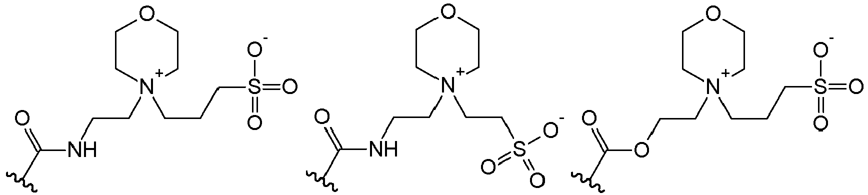 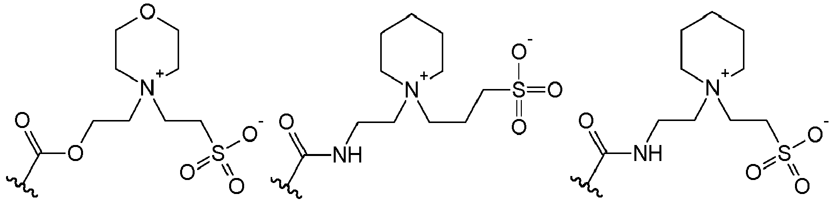 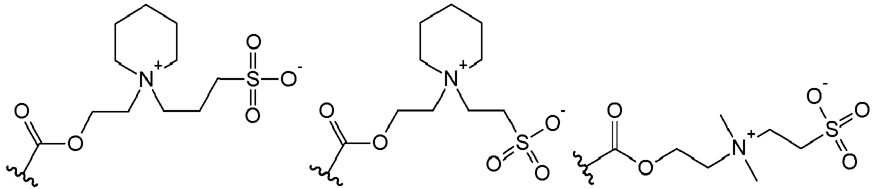 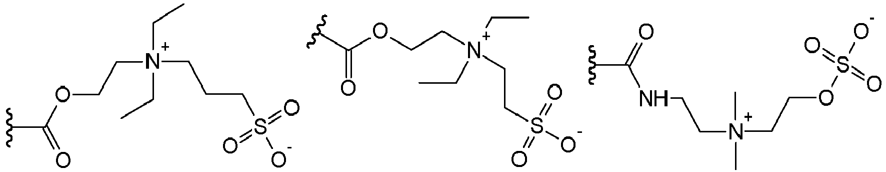 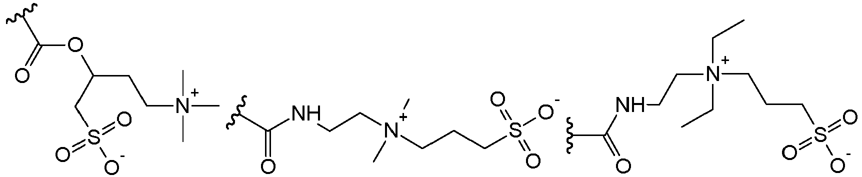 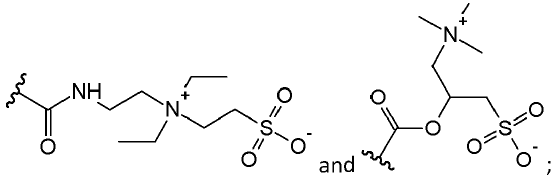 and
**R²** is selected from the group consisting of 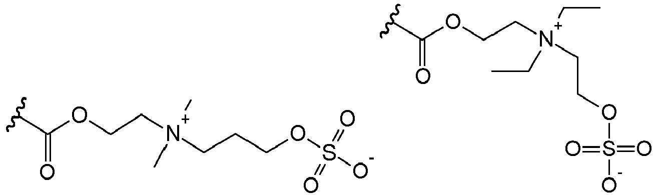 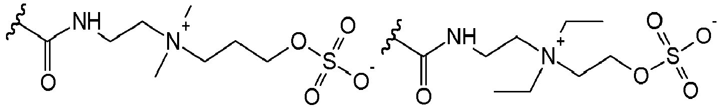 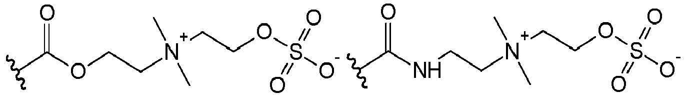 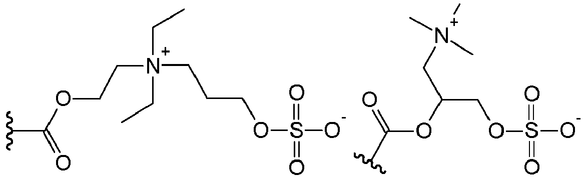 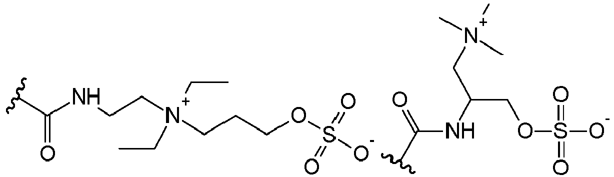 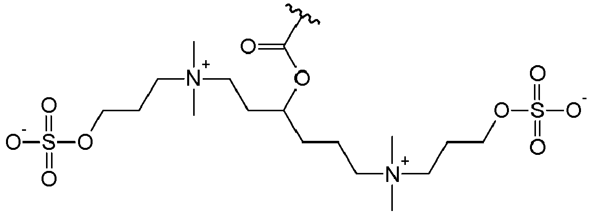 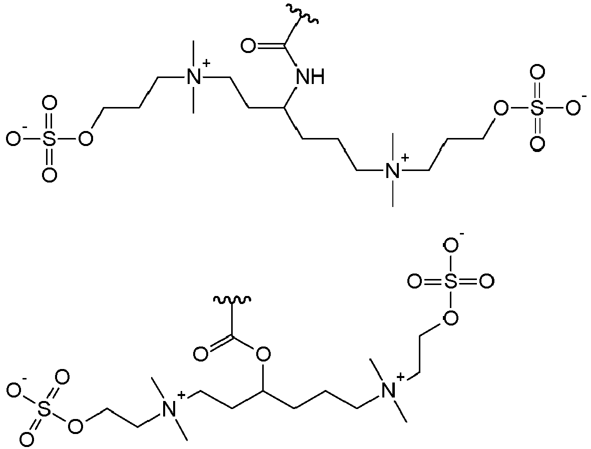 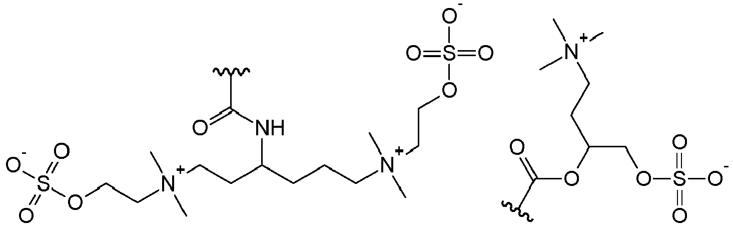 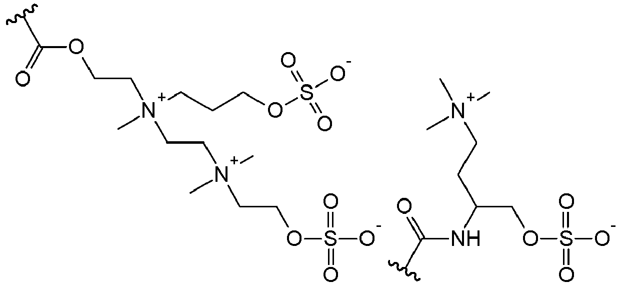 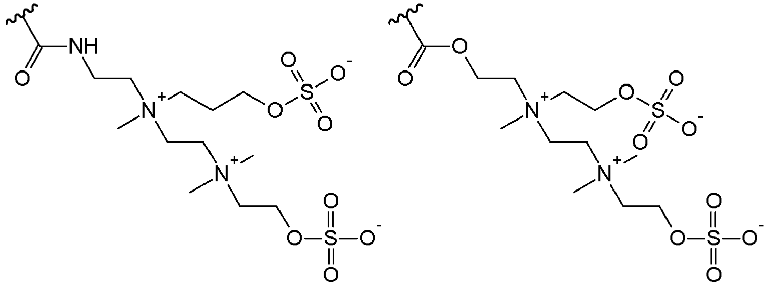 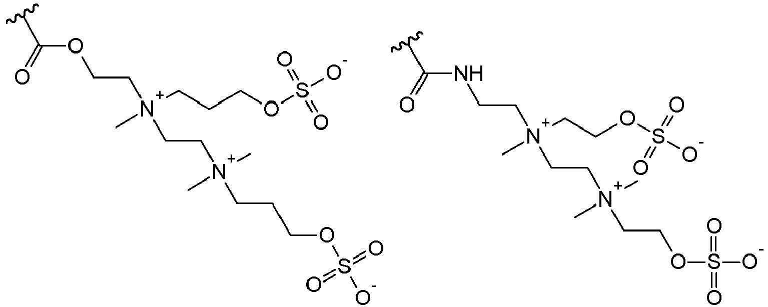 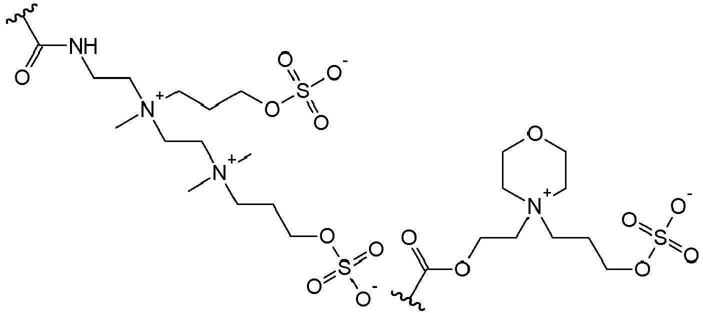 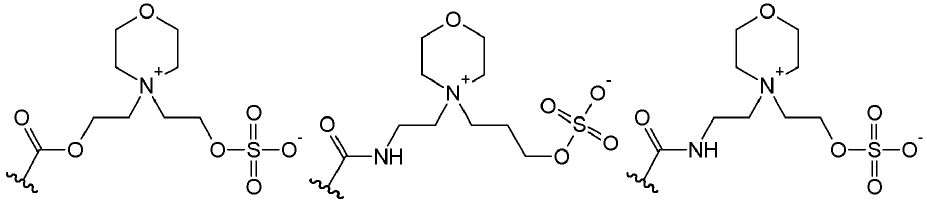 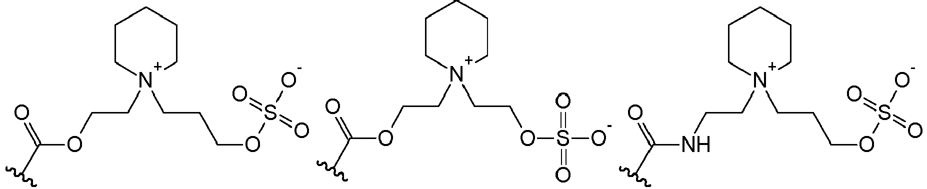 and

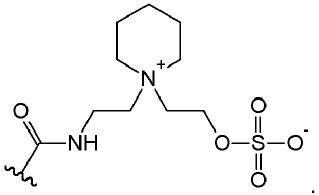

6. The use according to any of claims 1 to 5, wherein the polymer is selected from the group consisting of 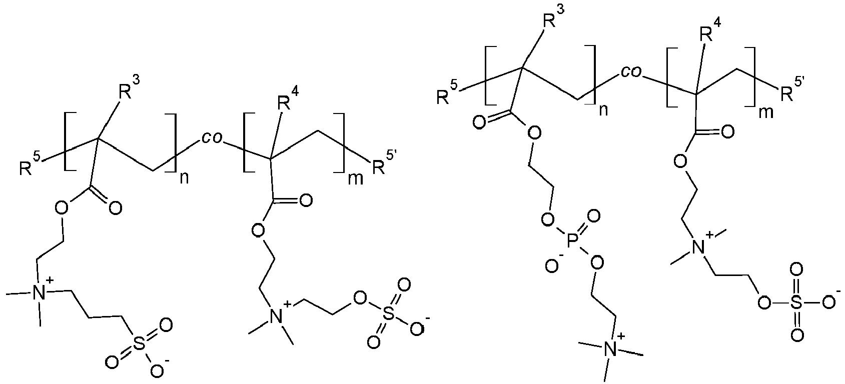 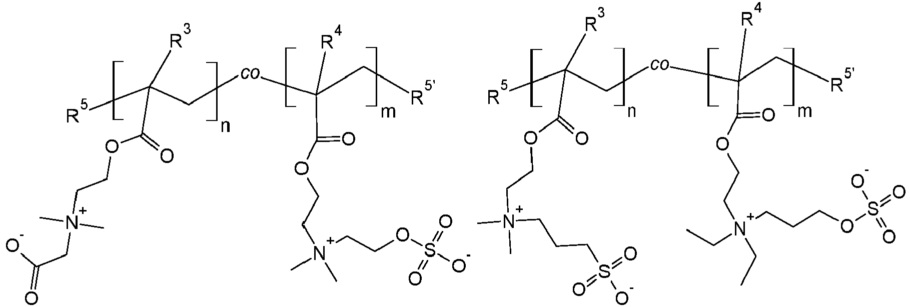 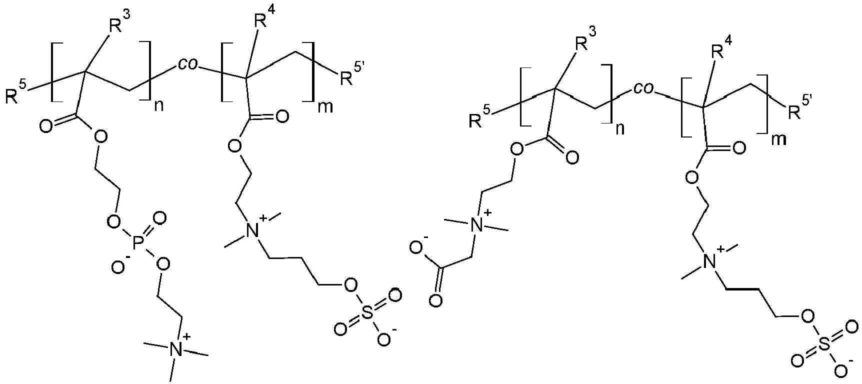 and

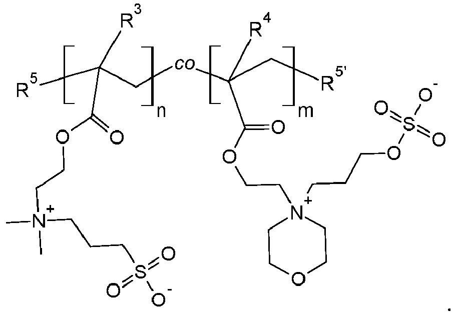

7. The use according to any of claims 1 to 6, wherein the crosslinker is selected from the group consisting of 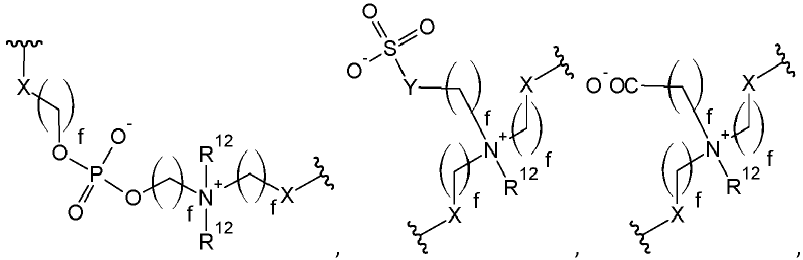 and 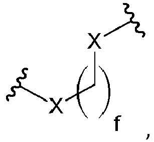 wherein
**f** is an integer selected from 1 to 5, optionally from 2 to 3;
**X** is selected from the group consisting of -C(=O)-O-, -C(=O)-NH-, aliphatic and aromatic carbo- or heterocycle, optionally -C(=O)-O- and -C(=O)-NH-;
**Y** is oxygen or absent; and
**R¹²** is selected from the group consisting of methyl, ethyl and propyl;
optionally the crosslinker is selected from the group consisting of

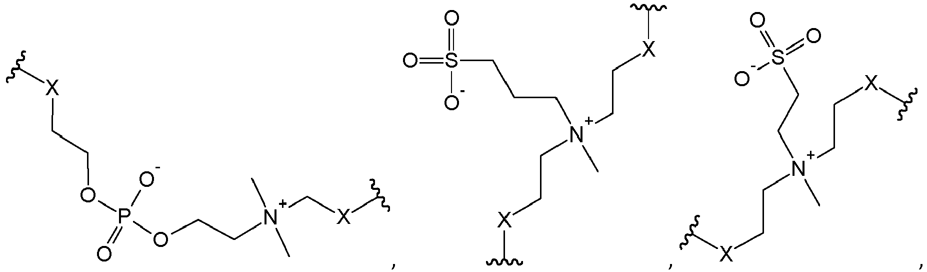

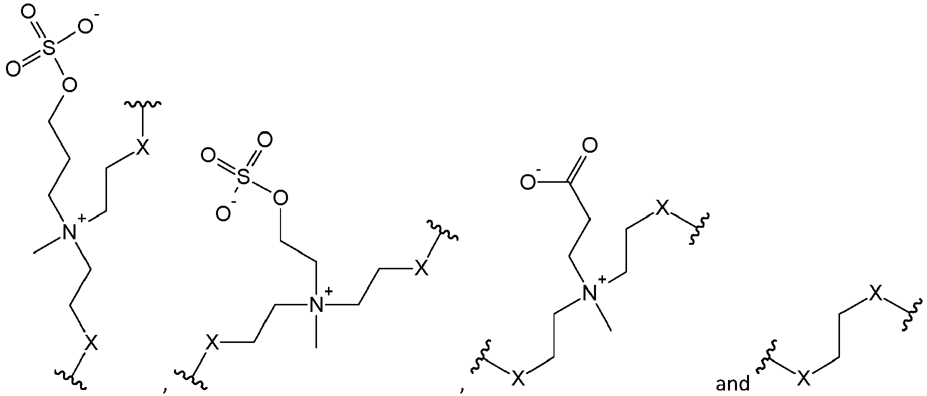

8. The use according to any of claims 1 to 7, wherein
a. the polymer has a degree of polymerization (n+m) from 5 to 5000, optionally from 50 to 500; and/or
b. the percentage of n in the polymer ([n/(n+m)]^{∗}100) is in the range from 0 to 99.5%, optionally 0 to 70%, optionally 20 to 60%; and/or
c. the dispersity of the polymer is in the range of 1 to 5, optionally 1 to 1.5;
d. the crosslinker molar fraction in the polymer is in the range of 0 to 99%, optionally 0 to 5%; and/or
e. the droplets are responsive to temperature, shear, ionic strength, pH and/or a magnetic field.

9. The use according to any of claims 1 to 8, wherein
a. the compounds for uptake, storage and/or reaction are selected from the group consisting of small molecules, drugs, antibodies, antigens, RNA, nucleic acids, viruses, carbohydrates, membrane-bound vesicles, contaminants, DNA, exosomes, extracellular vesicles, cells, proteins, peptides, biomolecules, enzymes, and ab42; and/or
b. the uptake is affinity- and/or binding-controlled, optionally mediated by interactions of the compound with R⁵ and/or R^{5'} of Formula (I).

10. The use according to any of claims 1 to 9, wherein the droplets are used
(i) for diagnosis of compounds, optionally of biomarkers,
(ii) for isolation for removal and/or enrichment of compounds, optionally for water treatment or cleaning,
(iii) for purification, extraction and/or separation of compounds,
(iv) for detoxification,
(v) for drug screening,
(vi) as cell culture scaffolds, and
(vii) in affinity assays.

11. A polymer for forming liquid droplets in an aqueous medium, wherein the polymer is a polymer as defined in any of claims 1 to 10 with the proviso that
(i) **R¹** is not 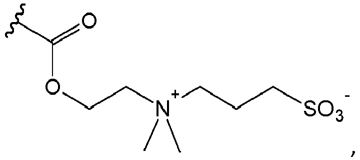 and/or
(ii) **R⁵** and **R**^{**5**'} are not 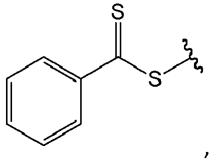 if **R²** is 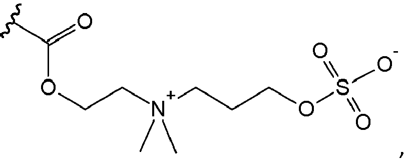 and wherein the droplets take up and/or store compounds in an aqueous medium.

12. The polymer according to claim 11, wherein **R¹** is selected from the group consisting of
(i) -X-hydrogen, -X-methyl, -X-ethyl and -X-propyl; 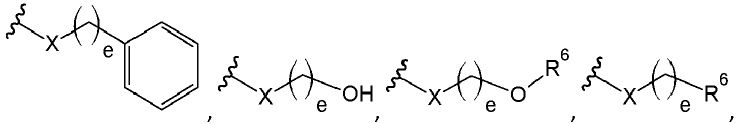
(ii) 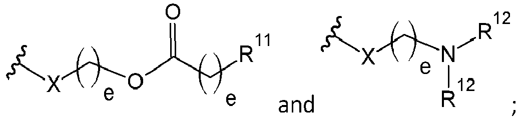
(iii) 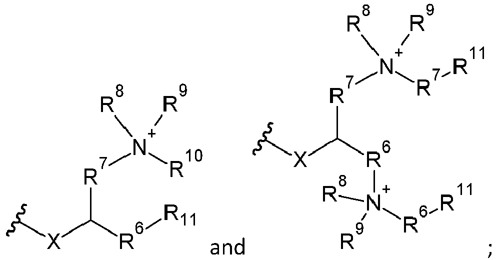
(iv) 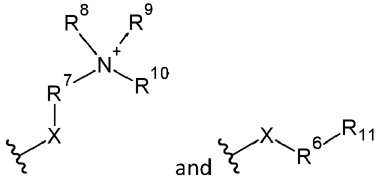 in a ratio of about 1:1 within the polymer;
(v) 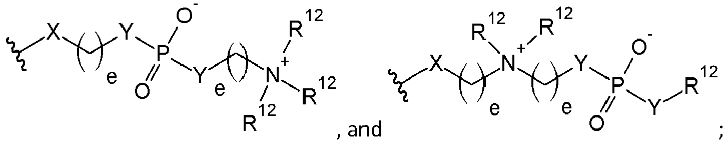 and 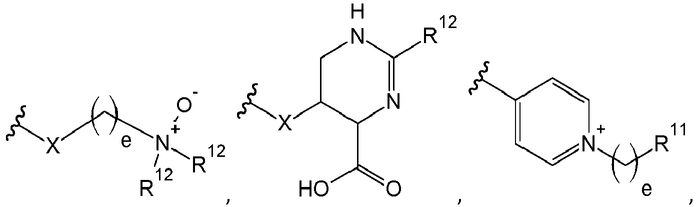
(vi)

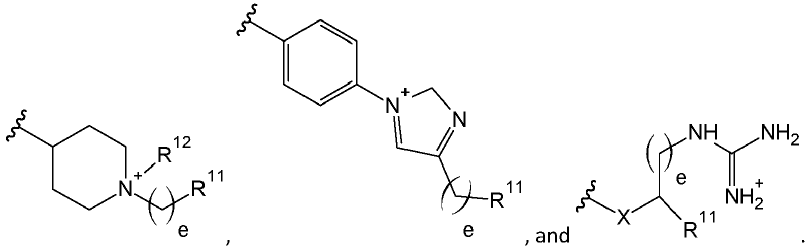

13. The polymer according to claim 11 or 12, wherein
**R¹** is selected from the group consisting of 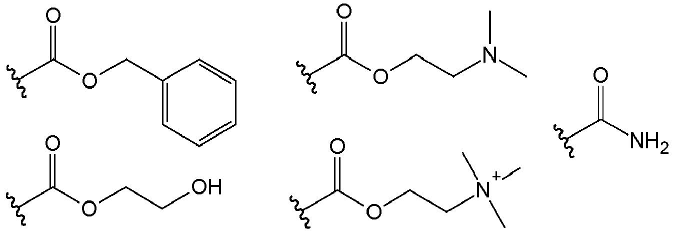 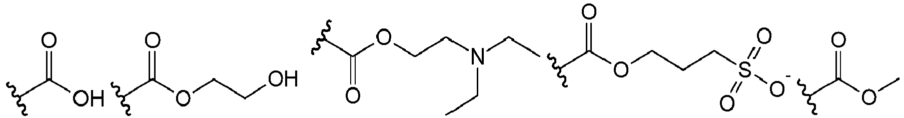 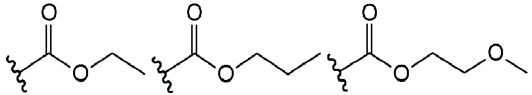 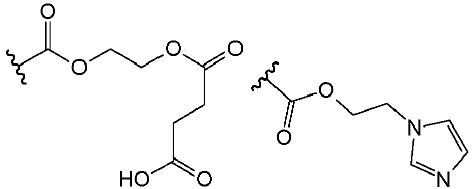 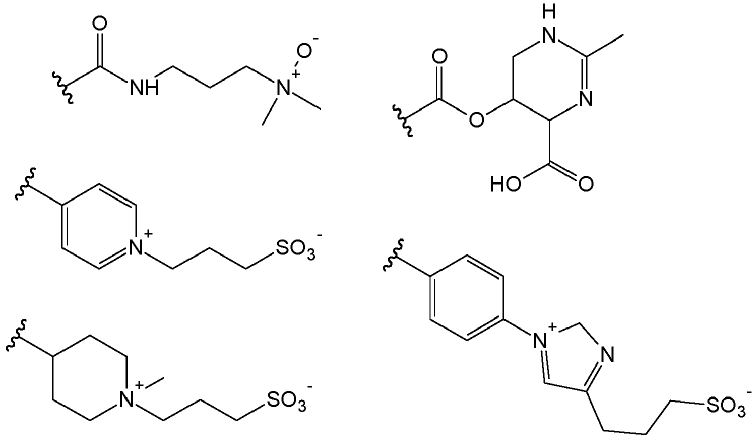 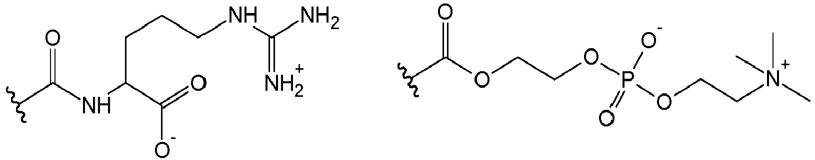 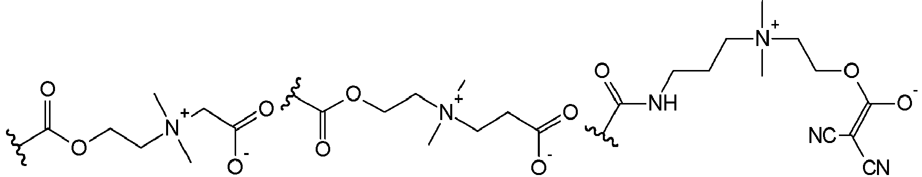 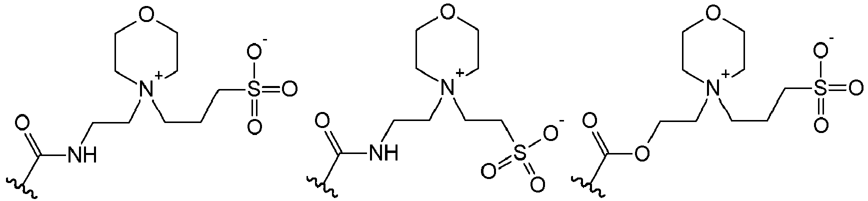 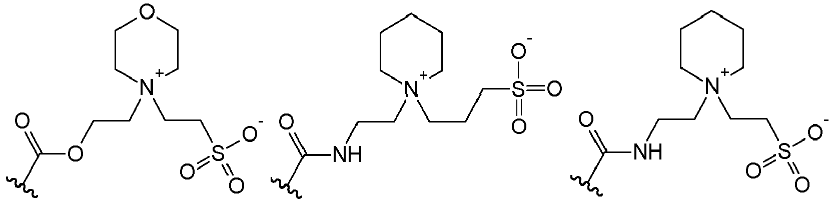 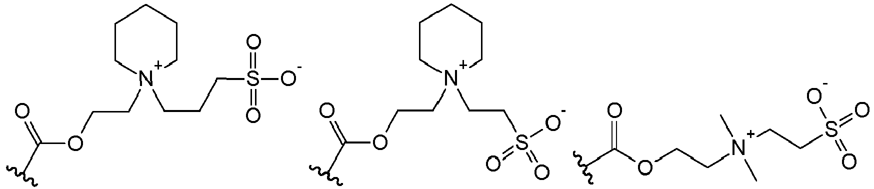 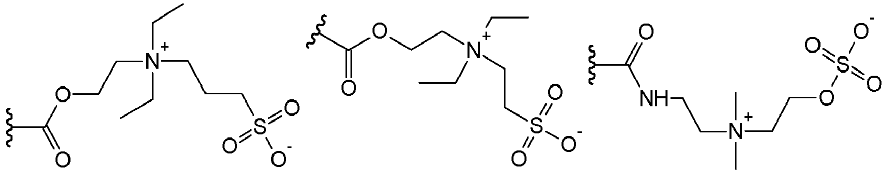 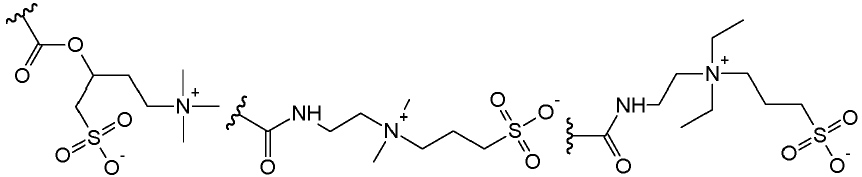 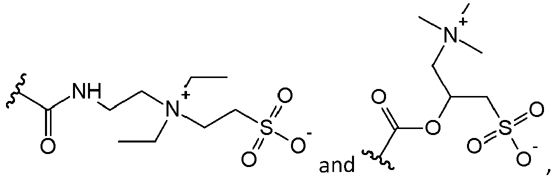 and/or
**R²** is selected from the group consisting of 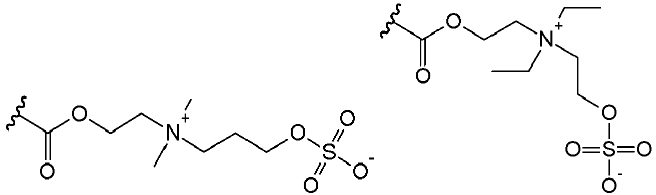 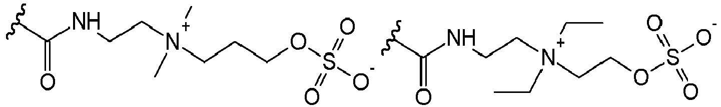 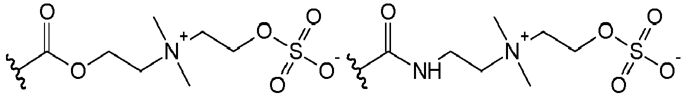 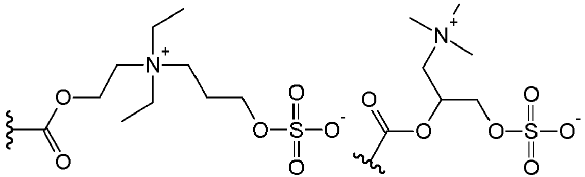 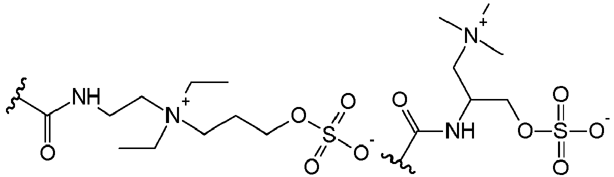 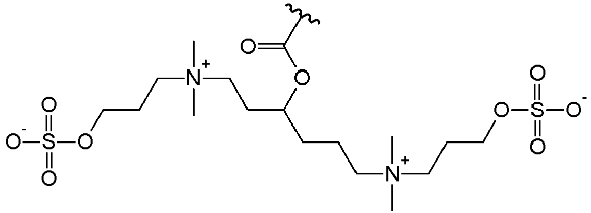 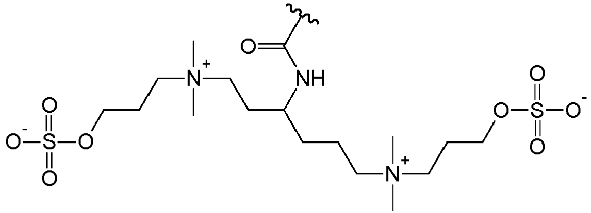 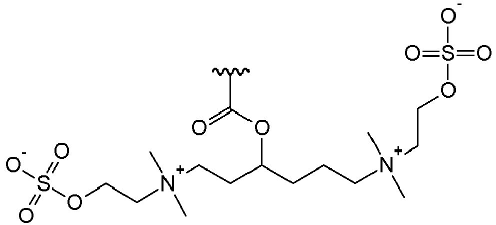 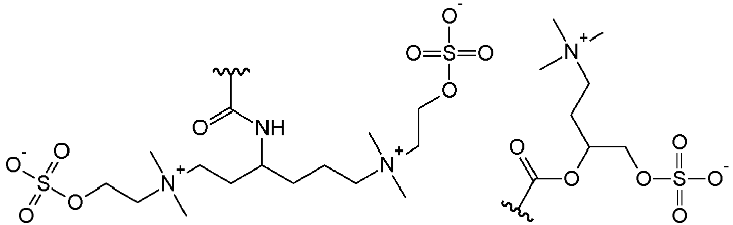 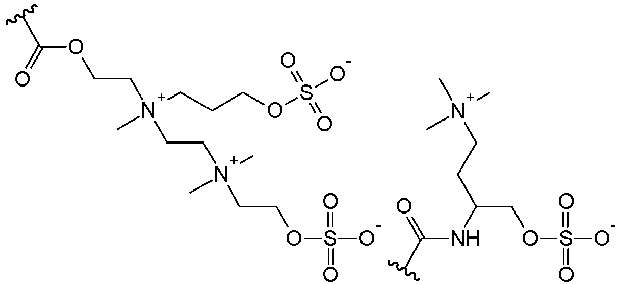 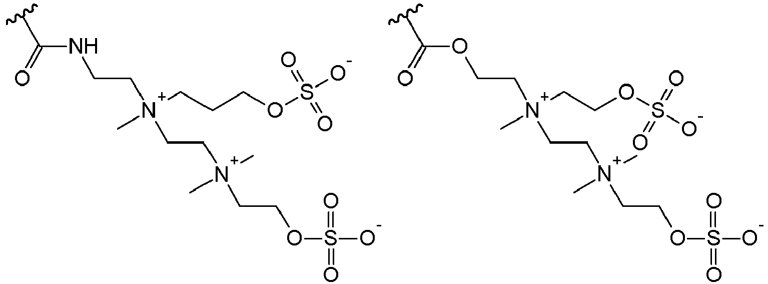 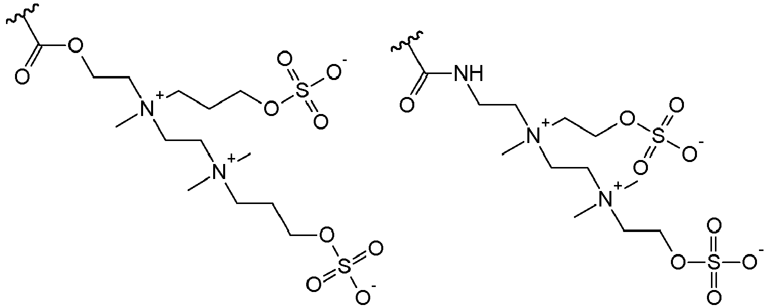 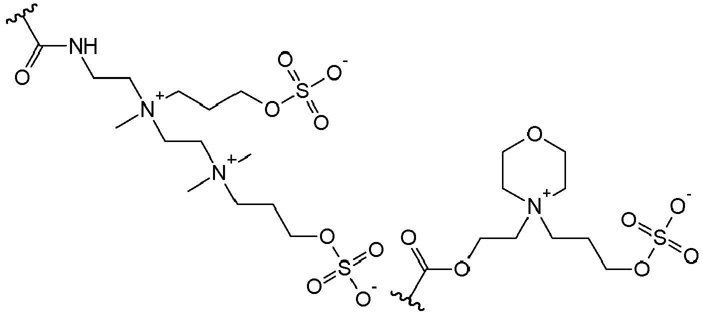 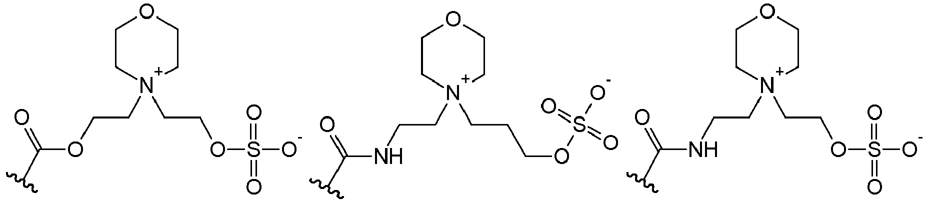 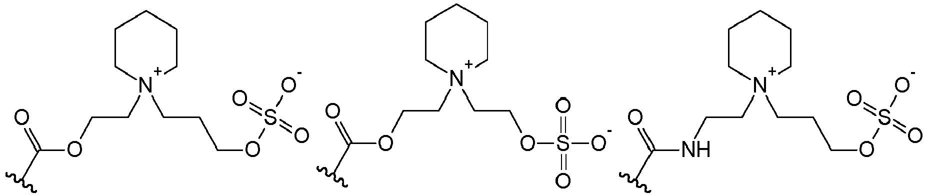 and

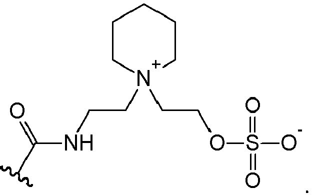

14. A method for producing a polymer according to any of claims 11 to 13 comprising the following steps:
(a) providing monomers selected from the group consisting of methyl methacrylate, ethyl methacrylate, propyl methacrylate, butyl methacrylate, hydroxyethyl methacrylate (HEMA), methacrylamide (Mam), benzyl methacrylate (BeMA), 2-dimethylaminoethyl methacrylate methyl chloride (MADQUAT) and 2-dimethylaminoethyl methacrylate (DMAEMA), 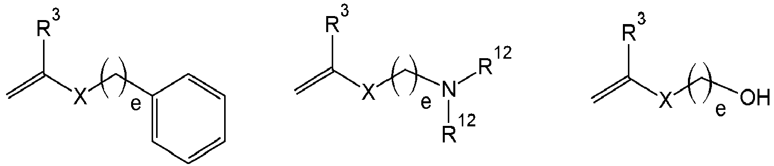 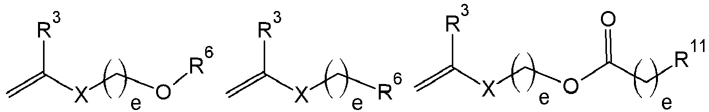 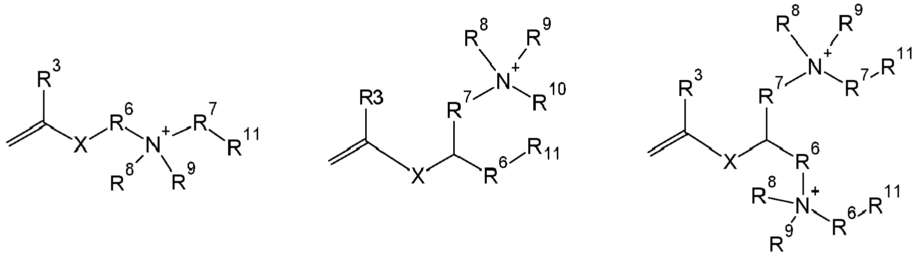 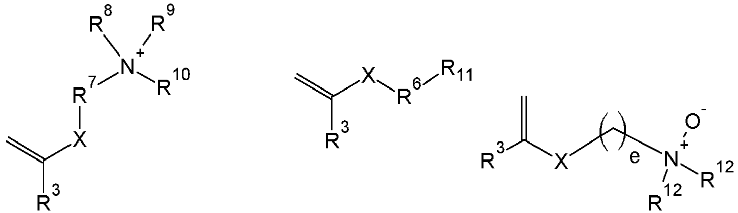 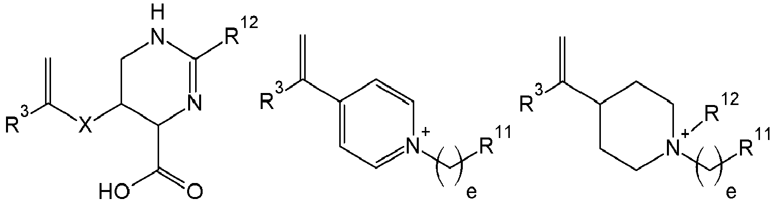 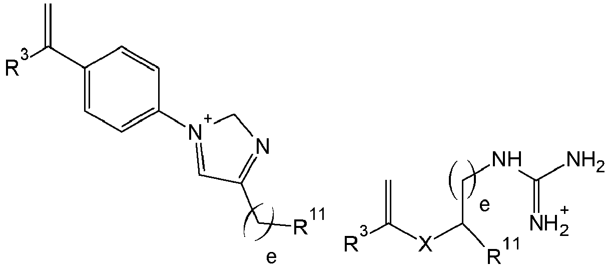 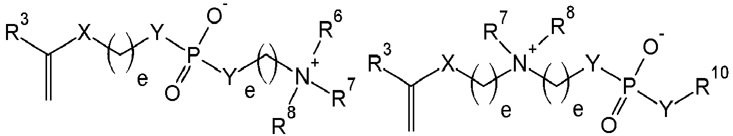 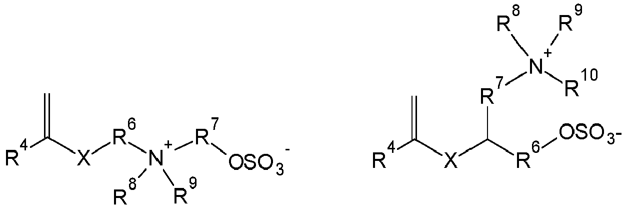 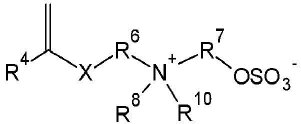 and 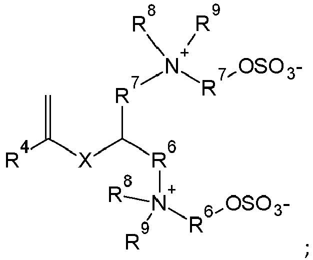
(b) optionally providing corsslinker monomers, optionally selected from the group consisting of 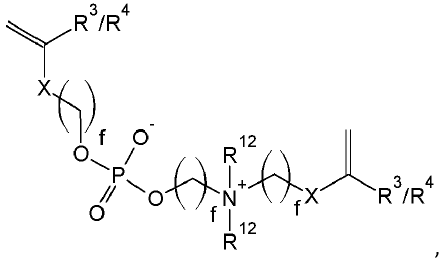 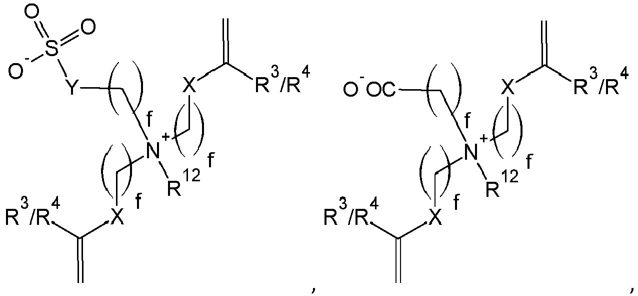 and 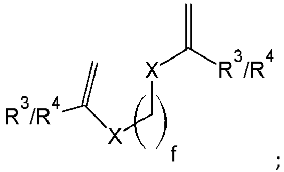
(c) reacting the monomers of step (a) and optionally (b) in a free radical polymerization or in a controlled radical polymerization, optionally using RAFT, ATRP or NMP in a suitable solvent;
(d) optionally functionalizing the polymer with or at R⁵ and/or R^{5'}, optionally in an esterification reaction, by click chemistry, amidation, thioesterification or by noncovalent modification; and
(e) isolating and optionally purifying the polymer.

15. A polymer for forming liquid droplets in an aqueous medium according to any of claims 11 to 13 obtained or obtainable by the process of claim 14.
